(19) Europäisches Patentamt / European Patent Office / Office européen des brevets

(11) **EP 4 205 739 A1**

(12) **EUROPEAN PATENT APPLICATION**
published in accordance with Art. 153(4) EPC

(43) Date of publication:
**05.07.2023 Bulletin 2023/27**

(21) Application number: **21845739.8**

(22) Date of filing: **22.07.2021**

(51) International Patent Classification (IPC):
***A61K 31/366*** $^{(2006.01)}$ ***A61K 31/357*** $^{(2006.01)}$
***A61K 31/541*** $^{(2006.01)}$ ***A61K 31/365*** $^{(2006.01)}$
***A61K 31/19*** $^{(2006.01)}$ ***A61K 31/137*** $^{(2006.01)}$
***A61K 9/00*** $^{(2006.01)}$ ***A61P 31/14*** $^{(2006.01)}$
***A61P 11/00*** $^{(2006.01)}$

(86) International application number:
**PCT/CN2021/107772**

(87) International publication number:
**WO 2022/017439 (27.01.2022 Gazette 2022/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.07.2020 CN 202010723800**

(71) Applicant: **Academy of Military Medical Sciences**
**Beijing 100850 (CN)**

(72) Inventors:
- **CAO, Ruiyuan**
  **Beijing 100850 (CN)**
- **WANG, Manli**
  **Beijing 100850 (CN)**
- **LI, Wei**
  **Beijing 100850 (CN)**
- **ZHAO, Lei**
  **Beijing 100850 (CN)**
- **YANG, Jingjing**
  **Beijing 100850 (CN)**
- **LI, Yuexiang**
  **Beijing 100850 (CN)**
- **FAN, Shiyong**
  **Beijing 100850 (CN)**
- **ZHOU, Xinbo**
  **Beijing 100850 (CN)**
- **XIAO, Dian**
  **Beijing 100850 (CN)**
- **HU, Zhihong**
  **Beijing 100850 (CN)**
- **LI, Song**
  **Beijing 100850 (CN)**
- **ZHONG, Wu**
  **Beijing 100850 (CN)**

(74) Representative: **Dey, Michael**
**Weickmann & Weickmann**
**Patent- und Rechtsanwälte PartmbB**
**Postfach 860 820**
**81635 München (DE)**

Remarks:
The complete document including Reference Table(s) and the Sequence Listing(s) can be downloaded from the EPO website

(54) **APPLICATION OF ARTEMISININ COMPOUND IN TREATMENT OF CORONAVIRUS INFECTION**

(57) The present invention relates to an application of an artemisinin compound in treatment of coronavirus infection. Specifically, the present invention provides an application of the compound, and a stereoisomer, a pharmaceutically acceptable salt, a solvate or a hydrate thereof in preparation of medicines. The medicines are used for treating diseases or infection caused by coronavirus (preferably SARS-CoV-2), and the compound is selected from one or more of artemisinin, arteether, artemether, artemisia ketone, dihydroartemisinin, artesunate, arteannuin B, and artemisinic acid.

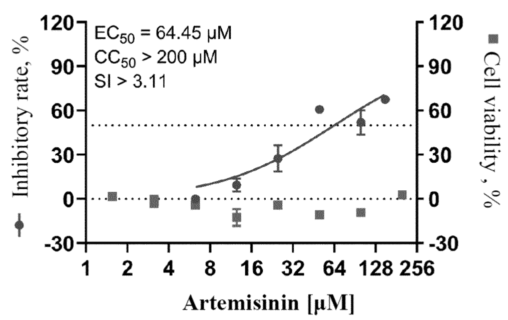


Figure 1

EP 4 205 739 A1

## Description

### Technical Field

The present invention relates to the field of biomedicine, in particular to the application of an artemisinin compound in the treatment of a coronavirus infection.

### Background Art

Artemisinin is a hemiterpene lactone compound extracted by Chinese scientists from compositae plant *Artemisia annua,* and artemisinin-related compounds (compounds of Formula I) are a group of compounds that are structurally similar to or in combination with artemisinin, and comprise arteether, artemether, artemisone, dihydroartemisinin, artesunate, arteannuin B (QHB), artemisinic acid and other compounds. Artemisinin and its derivatives are currently one of the most effective drugs for the treatment of malaria in the world, and artemisinin combination therapy has been widely used around the world. Among the artemisinin-related compounds, dihydroartemisinin, artemether, artesunate and arteether have all been marketed as antimalarial drugs. In addition, other derivatives such as arteannuin B and artemisinic acid also have good antimalarial activity. In addition to the treatment of malaria, artemisinin and its derivatives have also been shown to have various pharmacological activities such as antitumor, antifungal, antiinflammatory, antiviral, antifibrotic, and immunomodulatory effects.

The 2019 novel coronavirus (2019-nCoV) is a new strain of coronavirus that has never been found in humans before. On February 11, 2020, the International Committee on Taxonomy of Viruses (ICTV) announced that the official classification of the 2019 novel coronavirus (2019-nCoV) is severe acute respiratory syndrome coronavirus 2 (SARS-CoV-2). On the same day, the World Health Organization (WHO) announced that the official name of the disease caused by the virus is COVID-19. The symptoms of SARS-CoV-2 infection are mainly pneumonia, which can be divided into simple infection, mild pneumonia, severe pneumonia, acute respiratory distress syndrome, sepsis, and septic shock according to the severity of the disease. Patients with simple infection may have nonspecific symptoms such as fever, cough, sore throat, nasal congestion, fatigue, headache, muscle pain or discomfort, and elderly and immunosuppressed individuals may experience atypical symptoms. Patients with mild pneumonia mainly have symptoms of cough, dyspnea and tachypnea. Severe pneumonia can be seen in adolescents, adults, or children. And the main symptoms thereof are increased respiratory rate, severe respiratory failure or dyspnea, central cyanosis, lethargy, unconsciousness or convulsions, and gasp, etc. The lung images of acute respiratory distress syndrome are bilateral ground-glass opacities, which cannot be completely explained by effusion, lobar exudation, atelectasis, or pulmonary mass. And the main symptom of acute respiratory distress syndrome is pulmonary edema. Patients with sepsis often have fatal organ dysfunction, and patients with septic shock are the most critical patients with a high probability of death. At present, for the new coronavirus infection, supportive treatment is mainly used in clinic, and no specific antiviral drug is available.

### Contents of the present invention

The purpose of the present invention is to find a drug with antiviral activity against coronavirus, especially SARS-CoV-2, which can be used for related diseases caused by its infection, such as simple infection such as fever, cough and sore throat, pneumonia, acute or severe acute respiratory tract infection, hypoxic respiratory failure and acute respiratory distress syndrome, sepsis and septic shock, etc. In the present invention, it is found through creative research that artemisinin compounds (e.g., artemisinin, arteether, artemether, artemisone, dihydroartemisinin, artesunate, arteannuin B, artemisinic acid, preferably artemisinin, arteannuin B, artemether, artesunate, dihydroartemisinin, especially artemisinin) have a function of inhibiting the replication of SARS-CoV-2, and a good potential therapeutic effect in the treatment of a disease caused by SARS-CoV-2.

To this end, in the first aspect of the present invention, the present invention provides use of a compound, or a stereoisomer, a pharmaceutically acceptable salt, a solvate or a hydrate thereof in the manufacture of a medicament for the treatment of a disease or infection caused by a coronavirus (preferably SARS-CoV-2), and the compound is one or more selected from the group consisting of artemisinin, arteether, artemether, artemisone, dihydroartemisinin, artesunate, arteannuin B, and artemisinic acid.

The inventors found that artemisinin compounds (especially artemisinin) can inhibit viral replication on cells and reduce viral nucleic acid load in cell culture.

In some embodiments, the disease caused by SARS-CoV-2 is COVID-19.

In some embodiments, the disease or infection caused by SARS-CoV-2 is a respiratory disease, such as simple infection, mild pneumonia, severe pneumonia, acute respiratory infection, severe acute respiratory infection (SARI), hypoxic respiratory failure, acute respiratory distress syndrome, sepsis, septic shock, etc.

In some embodiments, the simple infection includes, but is not limited to, fever, cough, sore throat, nasal

congestion, fatigue, headache, muscle pain or discomfort. In some embodiments, the mild pneumonia includes, but is not limited to, cough, dyspnea, and/or tachypnea. In some embodiments, the severe pneumonia includes, but is not limited to, increased respiratory rate, severe respiratory failure or dyspnea, central cyanosis, lethargy, unconsciousness or convulsions, and gasp. In some embodiments, the acute respiratory distress syndrome includes, but is not limited to, pulmonary edema. In some embodiments, the sepsis includes, but is not limited to, organ dysfunction.

In some embodiments, the compound is one or more selected from the group consisting of artemisinin, arteannuin B, artemether, artesunate, and dihydroartemisinin.

In some embodiments, the compound is artemisinin, and its structure is

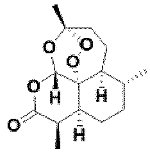

.

In the second aspect of the present invention, the present invention provides use of a pharmaceutical composition in the manufacture of a medicament for the treatment of a disease or infection caused by a coronavirus (preferably SARS-CoV-2), wherein the pharmaceutical composition comprises a compound, or a stereoisomer, a pharmaceutically acceptable salt, a solvate or a hydrate thereof, and the compound is one or more selected from the group consisting of artemisinin, arteether, artemether, artemisone, dihydroartemisinin, artesunate, arteannuin B, and artemisinic acid, preferably is one or more selected from the group consisting of artemisinin, arteannuin B, artemether, artesunate, and dihydroartemisinin, and more preferably is artemisinin.

In some embodiments, the disease caused by SARS-CoV-2 is COVID-19.

In some embodiments, the disease or infection caused by SARS-CoV-2 is a respiratory disease, such as simple infection, mild pneumonia, severe pneumonia, acute respiratory infection, severe acute respiratory infection (SARI), hypoxic respiratory failure, acute respiratory distress syndrome, sepsis, septic shock, etc.

In some embodiments, the simple infection includes, but is not limited to, fever, cough, sore throat, nasal congestion, fatigue, headache, muscle pain or discomfort. In some embodiments, the mild pneumonia includes, but is not limited to, cough, dyspnea and/or tachypnea. In some embodiments, the severe pneumonia includes, but is not limited to, increased respiratory rate, severe respiratory failure or dyspnea, central cyanosis, lethargy, unconsciousness or convulsions, and gasp. In some embodiments, the acute respiratory distress syndrome includes, but is not limited to, pulmonary edema. In some embodiments, the sepsis includes, but is not limited to, organ dysfunction.

In some embodiments, the pharmaceutical composition further comprises benflumetol.

In some embodiments, the pharmaceutical composition further comprises a pharmaceutically acceptable carrier or excipient, specifically, the pharmaceutical composition is a solid preparation, an injection, an external preparation, a spray, a liquid preparation, or a compound preparation.

In the third aspect of the present invention, the present invention provides a compound, or a stereoisomer, a pharmaceutically acceptable salt, a solvate or a hydrate thereof, for use in the treatment of a disease or infection caused by a coronavirus (preferably SARS-CoV-2), the compound is one or more selected from the group consisting of artemisinin, arteether, artemether, artemisone, dihydroartemisinin, artesunate, arteannuin B, and artemisinic acid.

In some embodiments, the disease caused by SARS-CoV-2 is COVID-19.

In some embodiments, the disease or infection caused by SARS-CoV-2 is a respiratory disease, such as simple infection, mild pneumonia, severe pneumonia, acute respiratory infection, severe acute respiratory infection (SARI), hypoxic respiratory failure, acute respiratory distress syndrome, sepsis, septic shock, etc.

In some embodiments, the simple infection includes, but is not limited to, fever, cough, sore throat, nasal congestion, fatigue, headache, muscle pain or discomfort. In some embodiments, the mild pneumonia includes, but is not limited to, cough, dyspnea and/or tachypnea. In some embodiments, the severe pneumonia includes, but is not limited to, increased respiratory rate, severe respiratory failure or dyspnea, central cyanosis, lethargy, unconsciousness or convulsions, and gasp. In some embodiments, the acute respiratory distress syndrome includes, but is not limited to, pulmonary edema. In some embodiments, the sepsis includes, but is not limited to, organ dysfunction.

In some embodiments, the compound is one or more selected from the group consisting of artemisinin, arteannuin B, artemether, artesunate, and dihydroartemisinin.

In some embodiments, the compound is artemisinin, and its structure is

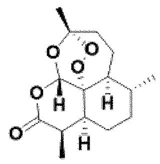

.

In the fourth aspect of the present invention, the present invention provides a pharmaceutical composition for use in treating a disease or infection caused by a coronavirus (preferably SARS-CoV-2), wherein the pharmaceutical composition comprises a compound, or a stereoisomer, a pharmaceutically acceptable salt, a solvate or a hydrate thereof, the compound is one or more selected from the group consisting of artemisinin, arteether, artemether, artemisone, dihydroartemisinin, artesunate, arteannuin B, and artemisinic acid, preferably is one or more selected from the group consisting of artemisinin, arteannuin B, artemether, artesunate, and dihydroartemisinin, more preferably is artemisinin.

In some embodiments, the disease caused by SARS-CoV-2 is COVID-19.

In some embodiments, the disease or infection caused by SARS-CoV-2 is a respiratory disease, such as simple infection, mild pneumonia, severe pneumonia, acute respiratory infection, severe acute respiratory infection (SARI), hypoxic respiratory failure, acute respiratory distress syndrome, sepsis, septic shock, etc.

In some embodiments, the simple infection includes, but is not limited to, fever, cough, sore throat, nasal congestion, fatigue, headache, muscle pain or discomfort. In some embodiments, the mild pneumonia includes, but is not limited to, cough, dyspnea and/or tachypnea. In some embodiments, the severe pneumonia includes, but is not limited to, increased respiratory rate, severe respiratory failure or dyspnea, central cyanosis, lethargy, unconsciousness or convulsions, and gasp. In some embodiments, the acute respiratory distress syndrome includes, but is not limited to, pulmonary edema. In some embodiments, the sepsis includes, but is not limited to, organ dysfunction.

In some embodiments, the pharmaceutical composition further comprises benflumetol.

In some embodiments, the pharmaceutical composition further comprises a pharmaceutically acceptable carrier or excipient, specifically, the pharmaceutical composition is a solid preparation, an injection, an external preparation, a spray, a liquid preparation, or a compound preparation.

In the fifth aspect of the present invention, the present invention provides a method for treating a disease, which comprises administering to a subject in need thereof a therapeutically effective amount of a compound, or a stereoisomer, a pharmaceutically acceptable salt, a solvate or a hydrate thereof, wherein the disease is a disease or infection caused by a coronavirus (preferably SARS-CoV-2), and the compound is one or more selected from the group consisting of artemisinin, arteether, artemether, artemisone, dihydroartemisinin, artesunate, arteannuin B, and artemisinic acid.

In some embodiments, the disease caused by SARS-CoV-2 is COVID-19.

In some embodiments, the disease or infection caused by SARS-CoV-2 is a respiratory disease, such as simple infection, mild pneumonia, severe pneumonia, acute respiratory infection, severe acute respiratory infection (SARI), hypoxic respiratory failure, acute respiratory distress syndrome, sepsis, septic shock, etc.

In some embodiments, the simple infection includes, but is not limited to, fever, cough, sore throat, nasal congestion, fatigue, headache, muscle pain or discomfort. In some embodiments, the mild pneumonia includes, but is not limited to, cough, dyspnea, and/or tachypnea. In some embodiments, the severe pneumonia includes, but is not limited to, increased respiratory rate, severe respiratory failure or dyspnea, central cyanosis, lethargy, unconsciousness or convulsions, and gasp. In some embodiments, the acute respiratory distress syndrome includes, but is not limited to, pulmonary edema. In some embodiments, the sepsis includes, but is not limited to, organ dysfunction.

In some embodiments, the compound is one or more selected from the group consisting of artemisinin, arteannuin B, artemether, artesunate, and dihydroartemisinin.

In some embodiments, the compound is artemisinin, and its structure is

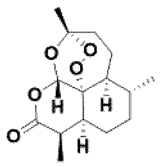

.

In the sixth aspect of the present invention, the present invention provides a method for treating a disease, which comprises administering to a subject in need thereof, a therapeutically effective amount of a pharmaceutical composition, wherein the disease is a disease or infection caused by a coronavirus (preferably SARS-CoV-2), the pharmaceutical composition comprises a compound, or a stereoisomer, a pharmaceutically acceptable salt, a solvate or a hydrate thereof, the compound is one or more selected from the group consisting of artemisinin, arteether, artemether,

artemisone, dihydroartemisinin, artesunate, arteannuin B, and artemisinic acid, preferably is one or more selected from the group consisting of artemisinin, arteannuin B, artemether, artesunate, and dihydroartemisinin, more preferably is artemisinin.

In some embodiments, the disease caused by SARS-CoV-2 is COVID-19.

In some embodiments, the disease or infection caused by SARS-CoV-2 is a respiratory disease, such as simple infection, mild pneumonia, severe pneumonia, acute respiratory infection, severe acute respiratory infection (SARI), hypoxic respiratory failure, acute respiratory distress syndrome, sepsis, septic shock, etc.

In some embodiments, the simple infection includes, but is not limited to, fever, cough, sore throat, nasal congestion, fatigue, headache, muscle pain or discomfort. In some embodiments, the mild pneumonia includes, but is not limited to, cough, dyspnea and/or tachypnea. In some embodiments, the severe pneumonia includes, but is not limited to, increased respiratory rate, severe respiratory failure or dyspnea, central cyanosis, lethargy, unconsciousness or convulsions, and gasp. In some embodiments, the acute respiratory distress syndrome includes, but is not limited to, pulmonary edema. In some embodiments, the sepsis includes, but is not limited to, organ dysfunction.

In some embodiments, the pharmaceutical composition further comprises benflumetol.

In some embodiments, the pharmaceutical composition further comprises a pharmaceutically acceptable carrier or excipient, specifically, the pharmaceutical composition is a solid preparation, an injection, an external preparation, a spray, a liquid preparation, or a compound preparation.

In the seventh aspect of the present invention, the present invention provides use of a compound, or a stereoisomer, a pharmaceutically acceptable salt, a solvate or a hydrate thereof in the manufacture of a medicament as an inhibitor of a coronavirus (preferably SARS-CoV-2), wherein the compound is one or more selected from the group consisting of artemisinin, arteether, artemether, artemisone, dihydroartemisinin, artesunate, arteannuin B, and artemisinic acid.

In some embodiments, the compound is one or more selected from the group consisting of artemisinin, arteannuin B, artemether, artesunate, and dihydroartemisinin.

In some embodiments, the compound is artemisinin, and its structure is

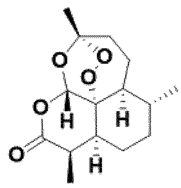

.

In the eighth aspect of the present invention, the present invention provides use of a pharmaceutical composition in the manufacture of a medicament as an inhibitor of a coronavirus (preferably SARS-CoV-2), wherein the pharmaceutical composition comprises a compound, or a stereoisomer, a pharmaceutically acceptable salt, a solvate or a hydrate thereof, the compound is one or more selected from the group consisting of artemisinin, arteether, artemether, artemisone, dihydroartemisinin, artesunate, arteannuin B, and artemisinic acid, preferably is one or more selected from the group consisting of artemisinin, arteannuin B, artemether, artesunate, and dihydroartemisinin, more preferably is artemisinin.

In some embodiments, the pharmaceutical composition further comprises benflumetol.

In some embodiments, the pharmaceutical composition further comprises a pharmaceutically acceptable carrier or excipient, specifically, the pharmaceutical composition is a solid preparation, an injection, an external preparation, a spray, a liquid preparation, or a compound preparation.

In the ninth aspect of the present invention, the present invention provides a compound, or a stereoisomer, a pharmaceutically acceptable salt, a solvate or a hydrate thereof, which is an inhibitor of a coronavirus (preferably SARS-CoV-2), wherein the compound is one or more selected from the group consisting of artemisinin, arteether, artemether, artemisone, dihydroartemisinin, artesunate, arteannuin B, and artemisinic acid.

In some embodiments, the compound is one or more selected from the group consisting of artemisinin, arteannuin B, artemether, artesunate, and dihydroartemisinin.

In some embodiments, the compound is artemisinin, and its structure is

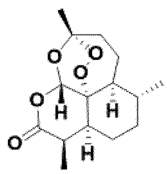

.

In the tenth aspect of the present invention, the present invention provides a pharmaceutical composition, which is an inhibitor of a coronavirus (preferably SARS-CoV-2), wherein the pharmaceutical composition comprises a compound, or a stereoisomer, a pharmaceutically acceptable salt, a solvate or a hydrate thereof, the compound is one or more selected from the group consisting of artemisinin, arteether, artemether, artemisone, dihydroartemisinin, artesunate, arteannuin B, and artemisinic acid, preferably is one or more selected from the group consisting of artemisinin, arteannuin B, artemether, artesunate, and dihydroartemisinin, more preferably is artemisinin.

In some embodiments, the pharmaceutical composition further comprises benflumetol.

In some embodiments, the pharmaceutical composition further comprises a pharmaceutically acceptable carrier or excipient, specifically, the pharmaceutical composition is a solid preparation, an injection, an external preparation, a spray, a liquid preparation, or a compound preparation.

In the eleventh aspect of the present invention, the present invention provides use of a compound, or a stereoisomer, a pharmaceutically acceptable salt, a solvate or a hydrate thereof in the manufacture of a medicament for inhibiting the replication or reproduction of a coronavirus (preferably SARS-CoV-2) in a cell (e.g., mammalian cell), wherein the compound is one or more selected from the group consisting of artemisinin, arteether, artemether, artemisone, dihydroartemisinin, artesunate, arteannuin B, and artemisinic acid.

In some embodiments, the compound is one or more selected from the group consisting of artemisinin, arteannuin B, artemether, artesunate, and dihydroartemisinin.

In some embodiments, the compound is artemisinin, and its structure is

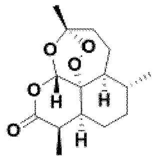

.

In the twelfth aspect of the present invention, the present invention provides use of a pharmaceutical composition in the manufacture of a medicament for inhibiting the replication or reproduction of a coronavirus (preferably SARS-CoV-2) in a cell (e.g., mammalian cell), wherein the pharmaceutical composition comprises a compound, or a stereoisomer, a pharmaceutically acceptable salt, a solvate or a hydrate thereof, the compound is one or more selected from the group consisting of artemisinin, arteether, artemether, artemisone, dihydroartemisinin, artesunate, arteannuin B, and artemisinic acid, preferably is one or more selected from the group consisting of artemisinin, arteannuin B, artemether, artesunate, and dihydroartemisinin, more preferably is artemisinin.

In some embodiments, the pharmaceutical composition further comprises benflumetol.

In some embodiments, the pharmaceutical composition further comprises a pharmaceutically acceptable carrier or excipient, specifically, the pharmaceutical composition is a solid preparation, an injection, an external preparation, a spray, a liquid preparation, or a compound preparation.

In the thirteenth aspect of the present invention, the present invention provides a compound, or a stereoisomer, a pharmaceutically acceptable salt, a solvate or a hydrate thereof, for use in inhibiting the replication or reproduction of a coronavirus (preferably SARS-CoV-2) in a cell (e.g., mammalian cell), wherein the compound is one or more selected from the group consisting of artemisinin, arteether, artemether, artemisone, dihydroartemisinin, artesunate, arteannuin B, and artemisinic acid.

In some embodiments, the compound is one or more selected from the group consisting of artemisinin, arteannuin B, artemether, artesunate, and dihydroartemisinin.

In some embodiments, the compound is artemisinin, its structure is

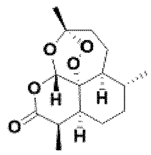

.

In the fourteenth aspect of the present invention, the present invention provides a pharmaceutical composition, for use in inhibiting the replication or reproduction of a coronavirus (preferably SARS-CoV-2) in a cell (e.g., mammalian cell), wherein the pharmaceutical composition comprises a compound, or a stereoisomer, a pharmaceutically acceptable salt, a solvate or a hydrate thereof, the compound is one or more selected from the group consisting of artemisinin, arteether, artemether, artemisone, dihydroartemisinin, artesunate, arteannuin B, and artemisinic acid, preferably is one

or more selected from the group consisting of artemisinin, arteannuin B, artemether, artesunate, and dihydroartemisinin, more preferably is artemisinin.

In some embodiments, the pharmaceutical composition further comprises benflumetol.

In some embodiments, the pharmaceutical composition further comprises a pharmaceutically acceptable carrier or excipient, specifically, the pharmaceutical composition is a solid preparation, an injection, an external preparation, a spray, a liquid preparation, or a compound preparation.

In the fifteenth aspect of the present invention, the present invention provides a method for inhibiting the replication or reproduction of a coronavirus (preferably SARS-CoV-2) in a cell (e.g., mammalian cell), which comprises administering to the cell (e.g., mammalian cell) an effective amount of a compound, or a stereoisomer, a pharmaceutically acceptable salt, a solvate or a hydrate thereof, wherein the compound is one or more selected from the group consisting of artemisinin, arteether, artemether, artemisone, dihydroartemisinin, artesunate, arteannuin B, and artemisinic acid.

In some embodiments, the compound is one or more selected from the group consisting of artemisinin, arteannuin B, artemether, artesunate, and dihydroartemisinin.

In some embodiments, the compound is artemisinin, and its structure is

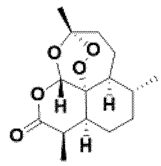

.

In the sixteenth aspect of the present invention, the present invention provides a method for inhibiting the replication or reproduction of a coronavirus (preferably SARS-CoV-2) in a cell (e.g., mammalian cell), which comprises administering to the cell (e.g., mammalian cell) an effective amount of a pharmaceutical composition, wherein the pharmaceutical composition comprises a compound, or a stereoisomer, a pharmaceutically acceptable salt, a solvate or a hydrate thereof, the compound is one or more selected from the group consisting of artemisinin, arteether, artemether, artemisone, dihydroartemisinin, artesunate, arteannuin B, and artemisinic acid, preferably is one or more selected from the group consisting of artemisinin, arteannuin B, artemether, artesunate, and dihydroartemisinin, more preferably is artemisinin.

In some embodiments, the pharmaceutical composition further comprises benflumetol.

In some embodiments, the pharmaceutical composition further comprises a pharmaceutically acceptable carrier or excipient, specifically, the pharmaceutical composition is a solid preparation, an injection, an external preparation, a spray, a liquid preparation, or a compound preparation.

In some embodiments, the mammal comprises bovine, equine, ovine, porcine, canine, feline, rodent, primate, such as human, cat, dog or pig.

In the present invention, the official classification name of the term "2019 novel coronavirus (2019-nCoV)" is called severe acute respiratory syndrome coronavirus 2 (SARS-CoV-2).

In the present invention, the official name of the term "disease caused by 2019 novel coronavirus (2019-nCoV)" is COVID-19.

In the present invention, "subject" refers to a vertebrate. In certain embodiments, the vertebrate refers to a mammal. The mammal comprises bovine, equine, ovine, porcine, canine, feline, rodent, primate, such as human, cat, dog or pig. Mammals include, but are not limited to, livestock (e.g., cattle), pet (e.g., cat, dog, and horse), primate, mouse and rat. In certain embodiments, the mammal refers to a human.

In the present invention, the term "therapeutically effective amount" or "prophylactically effective amount" refers to an amount that is sufficient to treat or prevent a patient’s disease but low enough to avoid serious side effects (at a reasonable benefit/risk ratio) within the scope of sound medical judgment. The therapeutically effective amount of a compound will depend on the particular compound selected (e.g., taking into account the potency, effectiveness and half-life of the compound), the route of administration selected, the disease being treated, the severity of the disease being treated, the age, size, weight and physical ailment of the patient being treated, the medical history of the patient being treated, duration of treatment, nature of concurrent therapy, desired therapeutic effect, and other factors, but can still be routinely determined by those skilled in the art.

In addition, it should be noted that the specific dosage and usage of the compound, its stereoisomer or its pharmaceutically acceptable salt and/or its solvate and/or its hydrate for different patients will depend on many factors, including the patient’s age, weight, gender, natural health status, nutritional status, the active strength of drug, the duration of administration, metabolic rate, the severity of condition, and the subjective judgment of treating physician. It is preferred here to use a dose between 0.001 and 1000 mg/kg body weight/day.

The pharmaceutically acceptable salt of the compound of the present invention comprises its inorganic or

organic acid salts, as well as inorganic or organic base salts, and the present invention relates to all forms of such salts, including but not limited to: sodium salt, potassium salt, calcium salt, lithium salt, meglumine salt, hydrochloride, hydrobromide, hydroiodide, nitrate, sulfate, disulfate, phosphate, diphosphate, acetate, propionate, butyrate, oxalate, trimethylacetate, adipate, alginate, lactate, citrate, tartrate, succinate, maleate, fumarate, picrate, aspartate, gluconate, benzoate, mesylate, ethanesulfonate, benzenesulfonate, p-toluenesulfonate and pamoate, etc.

The pharmaceutical composition involved in the present invention may comprises a pharmaceutically acceptable carrier, and the carrier includes but is not limited to: ion exchanger, alumina, aluminum stearate, lecithin, serum protein such as human serum albumin, buffer substance such as phosphate, glycerol, sorbic acid, potassium sorbate, partial glyceride mixture of saturated vegetable fatty acid, water, salt or electrolyte such as protamine sulfate, disodium hydrogen phosphate, potassium hydrogen phosphate, sodium chloride, zinc salt, colloidal silica, magnesium trisilicate, polyvinylpyrrolidone, cellulosic substance, polyethylene glycol, sodium carboxymethylcellulose, polyacrylate, beeswax, lanolin.

The pharmaceutical composition of the present invention can be prepared into various forms according to different administration routes.

According to the present invention, the pharmaceutical composition can be administered in any of the following ways: oral administration, spray inhalation, rectal administration, nasal administration, buccal administration, vaginal administration, topical administration, parenteral administration such as subcutaneous, intravenous, intramuscular, intraperitoneal, intrathecal, intraventricular, intrasternal and intracranial injection or infusion, or via an explanted reservoir. Of these, oral, intraperitoneal or intravenous administration is preferred.

For oral administration, the compound, its stereoisomer or its pharmaceutically acceptable salt and/or its solvate and/or its hydrate can be made into any orally acceptable preparation, including but not limited to tablet, capsule, aqueous solution or aqueous suspension. Among them, the commonly used carrier for tablet includes lactose and corn starch, and lubricant such as magnesium stearate may also be added. Commonly used diluent for capsule preparation includes lactose and dried cornstarch. Aqueous suspension is usually prepared by mixing the active ingredient with suitable emulsifying agent and suspending agent. If desired, some sweetening, flavoring or coloring agents may also be added to the above oral preparations.

For rectal administration, the compound, its stereoisomer or its pharmaceutically acceptable salt and/or its solvate and/or its hydrate can generally be made into the form of suppository, which is prepared by mixing the drug with a suitable non-irritating excipient. The excipient is solid at room temperature, but melts at rectal temperature to release the drug. Such excipient includes cocoa butter, beeswax and polyethylene glycol.

For topical administration, especially when treating affected surfaces or organs easily accessible by topical administration, such as eye, skin or lower intestinal neurological diseases, the compound, its stereoisomer or its pharmaceutically acceptable salt and/or its solvate and/or its hydrate can be made into different topical preparations according to different affected surfaces or organs, and the specific instructions are as follows:

For topical administration to the eye, the compound, its stereoisomer or its pharmaceutically acceptable salt and/or its solvate and/or its hydrate can be formulated into the form of a micronized suspension or solution, the carrier used is isotonic sterile saline with a certain pH, which may or may not be added with a preservative such as benzyl alkoxide chloride. In addition, for ophthalmic use, the compound can be formulated into the form of an ointment such as petrolatum ointment.

For topical administration to the skin, the compound, its stereoisomer or its pharmaceutically acceptable salt and/or its solvate and/or its hydrate can be made into the form of a suitable ointment, lotion or cream, in which the active ingredient is suspended or dissolved in one or more carriers. The carriers that can be used in ointment here include, but are not limited to: mineral oil, liquid petrolatum, white petrolatum, propylene glycol, polyethylene oxide, polypropylene oxide, emulsified wax and water; the carriers that can be used in lotion or cream include, but are not limited to: mineral oil, sorbitan monostearate, Tween 60, cetyl ester wax, cetenylaryl alcohol, 2-octyldodecanol, benzyl alcohol and water.

For topical administration to the lower intestinal tract, the compound, its stereoisomer or its pharmaceutically acceptable salt and/or its solvate and/or its hydrate can be made into the above-mentioned rectal suppository preparation or suitable form of enema preparation. In addition, topical transdermal patches can also be used.

The compound, its stereoisomer or its pharmaceutically acceptable salt and/or its solvate and/or its hydrate can also be administered in the form of sterile injectable preparation, including sterile injectable aqueous or oil suspension, or sterile injectable solution. Among them, the carriers and solvents that can be used are water, Ringer's solution and isotonic sodium chloride solution. In addition, sterile nonvolatile oil such as monoglyceride or diglyceride can also be used as a solvent or suspending medium.

The medicament of any one of the above-mentioned various dosage forms can be prepared according to the conventional methods in the pharmaceutical field.

## Brief Description of the Drawings

Figure 1 shows that artemisinin effectively reduced viral nucleic acid load in vero E6 cells infected with SARS-CoV-2. Artemisinin could inhibit the viral RNA load in the cells 48h after the cells were infected with SARS-CoV-2, and the inhibitory activity was dose-dependent. The left ordinate represents the percentage inhibition rate calculated based on the copy number of viral RNA in the sample (corresponding to the dots and their fitting line in the figure), the right ordinate represents the percentage toxicity calculated based on cell viability (corresponding to the squares and their fitting line in the figure), and the abscissa represents the concentration of drug (artemisinin).

Figure 2 shows that arteannuin B effectively reduced viral nucleic acid load in vero E6 cells infected with SARS-CoV-2. Arteannuin B could inhibit the viral RNA load in the cells 48h after the cells were infected with SARS-CoV-2, and the inhibitory activity was dose-dependent. The left ordinate represents the percentage inhibition rate calculated based on the copy number of viral RNA in the sample (corresponding to the dots and their fitting line in the figure), the right ordinate represents the percentage toxicity calculated based on cell viability (corresponding to the squares and their fitting line in the figure), and the abscissa represents the concentration of drug (arteannuin B).

Figure 3 shows that artemether effectively reduced viral nucleic acid load in vero E6 cells infected with SARS-CoV-2. Artemether could inhibit the viral RNA load in the cells 48h after the cells were infected with SARS-CoV-2, and the inhibitory activity was dose-dependent. The left ordinate represents the percentage inhibition rate calculated based on the copy number of viral RNA in the sample (corresponding to the dots and their fitting line in the figure), the right ordinate represents the percentage toxicity calculated based on cell viability (corresponding to the squares and their fitting line in the figure), and the abscissa represents the concentration of drug (artemether).

Figure 4 shows that artesunate effectively reduced viral nucleic acid load in vero E6 cells infected with SARS-CoV-2. Artesunate could inhibit the viral RNA load in the cells 48h after the cells were infected with SARS-CoV-2, and the inhibitory activity was dose-dependent. The left ordinate represents the percentage inhibition rate calculated based on the copy number of viral RNA in the sample (corresponding to the dots and their fitting line in the figure), the right ordinate represents the percentage toxicity calculated based on cell viability (corresponding to the squares and their fitting line in the figure), and the abscissa represents the concentration of drug (artesunate).

Figure 5 shows that dihydroartemisinin effectively reduced viral nucleic acid load in vero E6 cells infected with SARS-CoV-2. Dihydroartemisinin could inhibit the viral RNA load in the cells 48h after the cells were infected with SARS-CoV-2, and the inhibitory activity was dose-dependent. The left ordinate represents the percentage inhibition rate calculated based on the copy number of viral RNA in the sample (corresponding to the dots and their fitting line in the figure), the right ordinate represents the percentage toxicity calculated based on cell viability (corresponding to the squares and their fitting line in the figure), and the abscissa represents the concentration of drug (dihydroartemisinin).

## Specific Models for Carrying Out the present invention

The examples of the present invention will be described in detail below. The examples described below with reference to the accompanying drawings are exemplary, and are intended to be used to explain the present invention, but should not be construed as a limitation of the present invention.

The present invention provides a compound having the structure of Formula I, its stereoisomer, its pharmaceutically acceptable salt and/or its solvate and/or its hydrate:

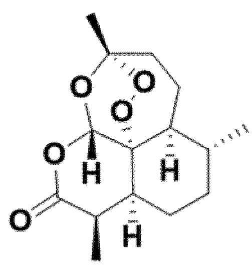

**Artemisinin**

Formula I

In certain embodiments, the pharmaceutically acceptable salts of the compound of Formula I of the present invention include inorganic or organic acid salts, and inorganic or organic base salts thereof, and the present invention relates to all forms of the above-mentioned salts, including but not limited to: sodium salt, potassium salt, calcium salt, lithium salt, meglumine salt, hydrochloride, hydrobromide, hydroiodide, nitrate, sulfate, disulfate, phosphate, diphosphate, acetate, propionate, butyrate, oxalate, trimethylacetate, adipate, alginate, lactate, citrate, tartrate, succinate, maleate, fumarate, picrate, aspartate, gluconate, benzoate, mesylate, ethanesulfonate, benzenesulfonate, p-toluenesulfonate and pamoate, etc.

The compound of Formula I can inhibit viral replication in cells and reduce viral nucleic acid load in cell culture.

The present invention relates to use of a compound represented by Formula I, its stereoisomer or its pharmaceutically acceptable salt and/or its solvate and/or its hydrate in the manufacture of a medicament for the treatment of a disease or infection (including but not limited to respiratory disease (e.g., simple infection such as fever, cough and sore throat, etc., pneumonia, acute or severe acute respiratory infection, hypoxic respiratory failure and acute respiratory distress syndrome, sepsis and septic shock, severe acute respiratory syndrome, etc.)) caused by a coronavirus, especially SARS-CoV-2,

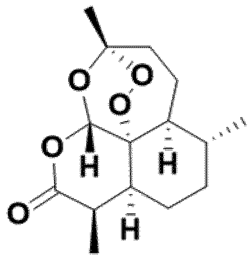

**Artemisinin**

I.

The present invention also relates to use of a compound represented by Formula I, its stereoisomer, its pharmaceutically acceptable salt and/or its solvate and/or its hydrate in the manufacture of a medicament as a coronavirus inhibitor.

The present invention also relates to use of a compound represented by Formula I, its stereoisomer, its pharmaceutically acceptable salt and/or its solvate and/or its hydrate in the manufacture of a medicament for inhibiting the replication or reproduction of a coronavirus in a cell (e.g., mammalian cell).

The present invention also relates to a pharmaceutical composition, which comprises a compound represented by the Formula I, its stereoisomer or its pharmaceutically acceptable salt and/or its solvate and/or its hydrate, preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

The present invention also relates to use of a pharmaceutical composition comprising a compound represented by the Formula I, its stereoisomer or its pharmaceutically acceptable salt and/or its solvate and/or its hydrate, or the compound represented by Formula I, its stereoisomer or its pharmaceutically acceptable salt and/or its solvate and/or its hydrate, in the manufacture of a medicament for the treatment of a disease including respiratory disease but not limited to respiratory disease (including simple infection, such as fever, cough and sore throat, etc., pneumonia, acute or severe acute respiratory tract infection, hypoxic respiratory failure and acute respiratory distress syndrome, sepsis and septic shock, severe acute respiratory syndrome, etc.).

The present invention also relates to a method for treating and/or preventing a disease in a mammal in need thereof or a method for inhibiting the replication or reproduction of a coronavirus in a mammal in need thereof, the method comprises administering to the mammal in need a therapeutically and/or prophylactically effective amount of the pharmaceutical composition comprising the compound represented by Formula I, its stereoisomer or its pharmaceutically acceptable salt and/or its solvate and/or its hydrate or the compound represented by Formula I, its stereoisomer, its pharmaceutically acceptable salt and/or its solvate and/or its hydrate, wherein the disease includes a disease caused by a coronavirus.

In certain embodiments, the disease caused by the coronavirus, especially SARS-CoV-2, includes but is not limited to respiratory disease (e.g., simple infection such as fever, cough and sore throat, etc., pneumonia, acute or severe acute respiratory tract infection, hypoxic respiratory failure and acute respiratory distress syndrome, sepsis and

septic shock, severe acute respiratory syndrome, etc.).

The present invention also relates to use of the pharmaceutical composition in the manufacture of a medicament for the treatment of a disease or infection (e.g., respiratory disease (e.g., simple infection such as fever, cough and sore throat, etc., pneumonia, acute or severe acute respiratory infection, hypoxic respiratory failure and acute respiratory distress syndrome, sepsis and septic shock, severe acute respiratory syndrome, etc.) caused by a coronavirus, especially SARS-CoV-2, wherein the pharmaceutical composition comprises the compound represented by Formula I, its stereoisomer or its pharmaceutically acceptable salt and/or its solvate and/or its hydrate

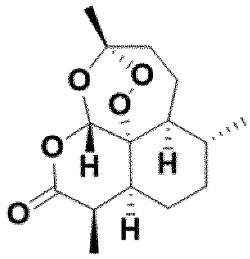

Artemisinin

I.

Preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

The present invention also relates to use of a pharmaceutical composition in the manufacture of a medicament as a coronavirus inhibitor, wherein the pharmaceutical composition comprises the compound represented by Formula I, its stereoisomer, its pharmaceutically acceptable salt and/or its solvate and/or its hydrate,

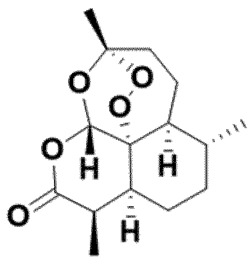

Artemisinin

I

Preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

The present invention also relates to use of a pharmaceutical composition in the manufacture of a medicament for inhibiting the replication or reproduction of a coronavirus in a cell (e.g., mammalian cell), wherein the pharmaceutical composition comprises a compound represented by Formula I, its stereoisomer, its pharmaceutically acceptable salt and/or its solvate and/or its hydrate,

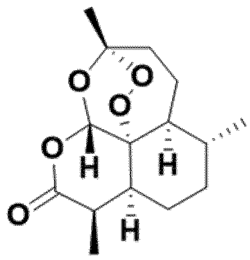

Artemisinin

I

Preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

The present invention also relates to a compound represented by Formula I, its stereoisomer or its pharmaceutically acceptable salt and/or its solvate and/or its hydrate, for use in the treatment of a disease or infection (including but not limited to respiratory disease (e.g., simple infection such as fever, cough and sore throat, etc., pneumonia, acute or severe acute respiratory infection, hypoxic respiratory failure and acute respiratory distress syndrome, sepsis or septic shock, severe acute respiratory syndrome, etc.)) caused by a coronavirus, especially SARS-CoV-2.

The present invention also relates to a compound represented by Formula I, its stereoisomer, its pharmaceutically acceptable salt and/or its solvate and/or its hydrate, which is used as a coronavirus inhibitor.

The present invention also relates to a compound represented by Formula I, its stereoisomer, its pharmaceutically acceptable salt and/or its solvate and/or its hydrate, for use in inhibiting the replication or reproduction of a coronavirus in a cell (e.g., mammalian cell).

The present invention also relates to a pharmaceutical composition for use in the treatment of a disease or infection (e.g., respiratory disease (e.g., simple infection such as fever, cough and sore throat, etc., pneumonia, acute or severe acute respiratory tract infection, hypoxic respiratory failure and acute respiratory distress syndrome, sepsis and septic shock, severe acute respiratory syndrome, etc.) caused by a coronavirus, especially SARS-CoV-2, wherein the pharmaceutical composition comprises a compound represented by the Formula I, its stereoisomer or its pharmaceutically acceptable salt and/or its solvate and/or its hydrate

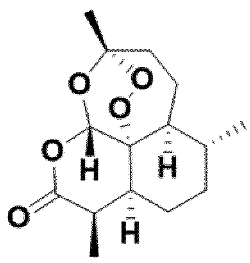

Artemisinin

I

Preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

The present invention also relates to a pharmaceutical composition, which is used as a coronavirus inhibitor, wherein the pharmaceutical composition comprises a compound represented by Formula I, its stereoisomer, its pharmaceutically acceptable salt and/or its solvate and/ or its hydrate,

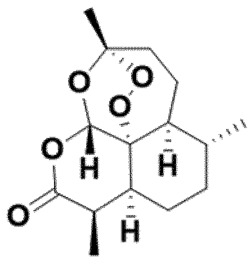

**Artemisinin**

I

Preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

The present invention also relates to a pharmaceutical composition, for use in inhibiting the replication or reproduction of a coronavirus in a cell (e.g., mammalian cells), wherein the pharmaceutical composition comprises a compound represented by Formula I, its stereoisomer, its pharmaceutically acceptable salt and/or its solvate and/or its hydrate,

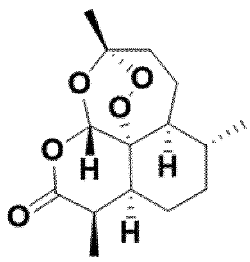

**Artemisinin**

I

Preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

In certain embodiments, the coronavirus of the present invention is SARS-CoV-2.

In certain embodiments, the disease caused by the coronavirus of the present invention is a disease caused by SARS-CoV-2, namely COVID-19.

In certain embodiments, the mammal of the present invention includes bovine, equine, ovine, porcine, canine, feline, rodent, primate, such as human, cat, dog or pig.

The present invention will be further explained below in conjunction with specific examples.

Example 1: Experiment of reducing viral nucleic acid load in SARS-CoV-2-infected cells by artemisinin

(1) Drug treatment of virus-infected cells

Vero E6 cells (purchased from ATCC, Cat. No. 1586) were inoculated into a 24-well plate and cultured for 24 hours; then virus infection was performed, specifically, SARS-CoV-2 (2019-nCoV) virus (nCoV-2019BetaCoV/Wuhan/WIV04/2019 strain, provided by Wuhan Institute of Virology, Chinese Academy of Sciences) was diluted to corresponding concentrations with 2% cell maintenance solution (formula: FBS (purchased from Gibco, Cat. No. 16000044) was added to MEM (purchased from Gibco, Cat. No. 10370021) at a volume ratio of 2% to form 2% cell maintenance solution), and then added to the 24-well plate so that each well contained 100 $TCID_{50}$ of virus. Next, artemisinin (purchased

from Selleck Chemicals, Cat. No. S1282) was diluted to corresponding concentrations with 2% cell maintenance solution and added to the corresponding wells, so that the final concentrations of the drug were 150 μM, 100 μM, 50 μM, 25 μM, 12.5μM, 6.25μM, respectively, and then the plate was allowed to stand in a 37°C, 5% $CO_2$ incubator for 48 hours, while 2% cell maintenance solution without any test drug was added only in the cell control group.

(2) RNA extraction

RNA extraction kit was purchased from Qiagen, Cat. No. 74106. The consumables (spin column, RNase-free 2mL collection tubes, etc.) and reagents (RLT, RW1, RPE, RNase-free water, etc.) involved in the following RNA extraction steps were all parts of the kit. The following extraction steps were all those recommended by the kit instructions.

1) 100 μL of the supernatant of the test culture plate was taken, added to a nuclease-free EP tube, then added with 350 μL of Buffer RLT, blown and mixed with a pipette gun to make it fully lysed, and then centrifuged to get a supernatant;

2) the supernatant obtained in 1) was added with an equal volume of 70% ethanol, and mixed well;

3) the mixture obtained in the 2) was transferred into an RNase-free spin column, centrifuged at 12,000 rpm for 15s, and the waste liquid was discarded;

4) 700 μL of Buffer RW1 was added to the spin column, centrifuged at 12,000 rpm for 15s to wash the spin column, and the waste liquid was discarded;

5) 500 μL of Buffer RPE was added to the spin column, centrifuged at 12000 rpm for 15s to wash the spin column, and the waste liquid was discarded;

6) 500 μL of Buffer RPE was added to the spin column, centrifuged at 12000 rpm for 2 min to wash the spin column, and the waste liquid was discarded;

7) a new 2mL RNase-free collection tube was used for placement of the spin column, centrifugation was performed at 12,000 rpm for 1 min, the spin column was dried, and then the entire spin column was transferred to the 1.5mL collection tube of step 8);

8) the spin column dried in step 7) was placed in a new 1.5mL collection tube, 30μL of RNase-free water was added to the spin column, centrifuged at 12,000 rpm for 2 minutes, and the eluate contained the corresponding RNA, RNase inhibitor (purchased from NEB company, Cat. No. M0314L) was added, and each RNA concentration was detected with Nano Drop (purchased from Thermo scientific, model Nano Drop One).

(3) RNA reverse transcription

In the experiment, the reverse transcription kit (PrimeScript™ RT reagent Kit with gDNA Eraser, Cat. No. RR047Q) produced by TaKaRa company was used for reverse transcription of RNA, and the steps were as follows.

① gDNA removal: RNA samples were collected from experimental groups, and 1 μg was taken from each sample for reverse transcription. First, 2 μL of 5× gDNA Eraser Buffer was added to the RNA of each experimental group, the reaction system was made up to 10 μL with RNase Free water, then the reaction system was mixed well, and placed in a water bath at 42°C for 2 min to remove the possible gDNA in the sample;

② reverse transcription: appropriate amounts of enzyme, primer Mix and reaction buffer were added to the sample obtained in ①, the volume was made up to 20 μL with RNase Free water, reaction was performed in a water bath at 37°C for 15 minutes, and then performed in a water bath at 85°C for 5 s, thereby obtaining cDNA by transcription.

(4) Real-time PCR

Fluorescence quantitative PCR was used to detect the number of copies per milliliter of the original virus solution.

The reaction system was mixed well using TB Green Premix (Takara, Cat#RR820A), and the amplification reaction and reading were performed in a StepOne Plus Real-time PCR machine (brand: ABI). The number of copies per milliliter of the original virus solution was calculated. The steps were as follows:

① Establishment of standard product: plasmid pMT-RBD (plasmid was provided by Wuhan Institute of Virology, Chinese Academy of Sciences) was diluted to $5\times10^8$ copies/μL, $5\times10^7$ copies/μL, $5\times10^6$ copies/μL, $5\times10^5$ copies/μL, $5\times10^4$ copies/μL, $5\times10^3$ copies/μL, $5\times10^2$ copies/μL. 2 μL of standard or cDNA template was taken and used for qPCR reaction.

② The primer sequences used in the experiment were as follows (all were indicated in the 5'-3' direction):

RBD-qF: CAATGGTTTAACAGGCACAGG (SEQ ID NO: 1)

RBD-qR: CTCAAGTGTCTGTGGATCACG (SEQ ID NO: 2)

③ The reaction procedure was as follows:

pre-denaturation: 95°C for 5 minutes;

cycling parameters: 95°C for 15 seconds, 54°C for 15 seconds, and 72°C for 30 seconds, a total of 40 cycles.

(5) Detection of drug cytotoxicity

The detection of drug cytotoxicity was performed by CCK-8 kit (Beoytime). Specific steps were as follows:

① $1\times10^4$ Vero E6 (ATCC) cells were inoculated into a 96-well plate and cultured at 37°C for 8 hours.

② The test drug was diluted with DMSO to achieve an appropriate stock solution concentration, and then diluted with MEM medium (purchased from Gibco, Cat. No. 10370021) containing 2% FBS (purchased from Gibco, Cat. No. 16000044) to the same concentration as that for the drug treatment. The original medium in the 96-well plate was discarded, 100 μL of drug-containing MEM medium was added to the cells, and three replicate wells were made for each concentration. Negative controls (adding DMSO and medium to the cell wells without adding drug) and blank controls (no cells, adding DMSO and medium) were set. After the drug was added, the cells were cultured at 37°C for 48 hours.

③ 20 μL of CCK-8 solution (Beoytime) was added to the wells to be tested, mixed gently without generating air bubbles, and continued to incubate at 37°C for 2 hours. $OD_{450}$ was read on a microplate reader (purchased from Molecular Devices, model SpectraMax M5), and cell viability was calculated:

$$\text{Cell viability (\%)} = (A_{\text{(drug treatment group)}} - A_{\text{(blank control)}}) / (A_{\text{(negative control)}} - A_{\text{(blank control)}}) \times 100\%$$

wherein A represents reading of microplate reader.

(6) Experimental results

The results of the virus proliferation inhibition experiment showed that the test compound could effectively inhibit the replication of SARS-CoV-2 virus genome in the infection supernatant at concentrations of 150 μM, 100 μM and 50 μM. (Table 1 and Figure 1)

Table 1. Antiviral experiment of test compound (artemisinin)

| Concentration (μM) | 150 | 100 | 50 | 25 | 12.5 | 6.25 | Vehicle |
|---|---|---|---|---|---|---|---|
| Number of copies of viral genome (MOI = 0.05) | 25440652 ± 1521221 | 37237188 ± 3256825 | 31079912 ± 6766848 | 61918172 ± 24491538 | 72012135 ± 16340753 | 79058074 ± 12572555 | 78924174 ± 25543666 |

The cytotoxicity results showed that the treatment of the test compound (artemisinin) did not change the cell

viability at all the tested concentrations, that was, the test compound had no toxic effect on the cells at all concentrations (Table 2 and Figure 1).

Table 2. Cytotoxicity test of test compound (artemisinin)

| Concentration (μM) | 200 | 100 | 50 | 25 | 12.5 | 6.25 | 3.13 | 1.56 | Vehicle |
|---|---|---|---|---|---|---|---|---|---|
| Cell viability (% of negative control) | 97.14 ± 4.23 | 109.14 ± 3.64 | 110.71 ± 2.04 | 104.26 ± 2.98 | 112.55 ± 9.91 | 104.23 ± 4.25 | 101.55 ± 6.44 | 98.45 ± 4.39 | 100 ± 2.56 |

Example 2: Experiment of reducing viral nucleic acid load in SARS-CoV-2-infected cells by arteannuin B

(1) Drug treatment of virus-infected cells

Vero E6 cells (purchased from ATCC, Cat. No. 1586) were inoculated into a 24-well plate and cultured for 24 hours; then virus infection was performed, specifically, SARS-CoV-2 (2019-nCoV) virus (nCoV-2019BetaCoV/Wuhan/WIV04/2019 strain, provided by Wuhan Institute of Virology, Chinese Academy of Sciences) was diluted to corresponding concentrations with 2% cell maintenance solution (formula: FBS (purchased from Gibco, Cat. No. 16000044) was added to MEM (purchased from Gibco, Cat. No. 10370021) at a volume ratio of 2% to form 2% cell maintenance solution), and then added to the 24-well plate so that each well contained 100 $TCID_{50}$ of virus. Next, arteannuin B (purchased from MCE, Cat. No. HY-N2016) was diluted to corresponding concentrations with 2% cell maintenance solution and added to the corresponding wells, so that the final concentrations of the drug were 50 μM, 25 μM, 12.5 μM, 6.25μM, 3.13μM, 1.56μM, respectively, and then the plate was allowed to stand in a 37°C, 5% $CO_2$ incubator for 48 hours, while 2% cell maintenance solution without any test drug was added only in the cell control group.

(2) RNA extraction

RNA extraction kit was purchased from Qiagen, Cat. No. 74106. The consumables (spin column, RNase-free 2mL collection tubes, etc.) and reagents (RLT, RW1, RPE, RNase-free water, etc.) involved in the following RNA extraction steps were all parts of the kit. The following extraction steps were all those recommended by the kit instructions.

1) 100 μL of the supernatant of the test culture plate was taken, added into a nuclease-free EP tube, then added with 350 μL of Buffer RLT, blown and mixed with a pipette gun to make it fully lysed, and then centrifuged to get a supernatant;

2) the supernatant obtained in 1) was added with an equal volume of 70% ethanol, and mixed well;

3) the mixture obtained in the 2) was transferred into an RNase-free spin column, centrifuged at 12,000 rpm for 15s, and the waste liquid was discarded;

4) 700 μL of Buffer RW1 was added to the spin column, centrifuged at 12,000 rpm for 15s to wash the spin column, and the waste liquid was discarded;

5) 500 μL of Buffer RPE was added to the spin column, centrifuged at 12000 rpm for 15s to wash the spin column, and the waste liquid was discarded;

6) 500 μL of Buffer RPE was added to the spin column, centrifuged at 12000 rpm for 2 min to wash the spin column, and the waste liquid was discarded;

7) a new 2mL RNase-free collection tube was used for placement of the spin column, centrifugation was performed at 12,000 rpm for 1 min, the spin column was dried, and then the entire spin column was transferred to the 1.5mL collection tube of step 8);

8) the spin column dried in step 7) was placed in a new 1.5mL collection tube, 30μL of RNase-free water was added to the spin column, centrifuged at 12,000 rpm for 2 minutes, and the eluate contained the corresponding RNA, RNase inhibitor (purchased from NEB company, Cat. No. M0314L) was added, and each RNA concentration was detected with Nano Drop (purchased from Thermo scientific, model Nano Drop One).

(3) RNA reverse transcription

In the experiment, the reverse transcription kit (PrimeScript™ RT reagent Kit with gDNA Eraser, Cat. No. RR047Q) produced by TaKaRa company was used for reverse transcription of RNA, and the steps were as follows.

① gDNA removal: RNA samples were collected from experimental groups, and 1 μg was taken from each sample for reverse transcription. First, 2 μL of 5× gDNA Eraser Buffer was added to the RNA of each experimental group, the reaction system was made up to 10 μL with RNase Free water, then the reaction system was mixed well, and placed in a water bath at 42°C for 2 min to remove the possible gDNA in the sample;

② reverse transcription: appropriate amounts of enzyme, primer Mix and reaction buffer were added to the sample obtained in ①, the volume was made up to 20 μL with RNase Free water, reaction was performed in a water bath at 37°C for 15 minutes, and then performed in a water bath at 85°C for 5 s, thereby obtaining cDNA by transcription.

(4) Real-time PCR

Fluorescence quantitative PCR was used to detect the number of copies per milliliter of the original virus solution.

The reaction system was mixed well using TB Green Premix (Takara, Cat#RR820A), and the amplification reaction and reading were performed in a StepOne Plus Real-time PCR machine (brand: ABI). The number of copies per milliliter of the original virus solution was calculated. The steps were as follows:

① Establishment of standard product: plasmid pMT-RBD (plasmid was provided by Wuhan Institute of Virology, Chinese Academy of Sciences) was diluted to $5\times10^8$ copies/μL, $5\times10^7$ copies/μL, $5\times10^6$ copies/μL, $5\times10^5$ copies/μL, $5\times10^4$ copies/μL, $5\times10^3$ copies/μL, $5\times10^2$ copies/μL. 2 μL of standard or cDNA template was taken and used for qPCR reaction.

② The primer sequences used in the experiment were as follows (all were indicated in the 5'-3' direction):

RBD-qF: CAATGGTTTAACAGGCACAGG (SEQ ID NO: 1)

RBD-qR: CTCAAGTGTCTGTGGATCACG (SEQ ID NO: 2)

③ The reaction procedure was as follows:

pre-denaturation: 95°C for 5 minutes;

cycling parameters: 95°C for 15 seconds, 54°C for 15 seconds, and 72°C for 30 seconds, a total of 40 cycles.

(5) Detection of drug cytotoxicity

The detection of drug cytotoxicity was performed by CCK-8 kit (Beoytime). Specific steps were as follows:

① $1\times10^4$ Vero E6 (ATCC) cells were inoculated into a 96-well plate and cultured at 37°C for 8 hours.

② The test drug was diluted with DMSO to achieve an appropriate stock solution concentration, and then diluted with MEM medium (purchased from Gibco, Cat. No. 10370021) containing 2% FBS (purchased from Gibco, Cat. No. 16000044) to the same concentration as that for the drug treatment. The original medium in the 96-well plate was discarded, 100 μL of the drug-containing MEM medium was added to the cells, and three replicate wells were made for each concentration. Negative controls (adding DMSO and medium to the cell wells without adding drug) and blank controls (no cells, adding DMSO and medium) were set. After the drug was added, the cells were cultured at 37°C for 48 hours.

③ 20 μL of CCK-8 solution (Beoytime) was added to the wells to be tested, mixed gently without generating air bubbles, and continued to incubate at 37°C for 2 hours. $OD_{450}$ was read on a microplate reader (purchased from Molecular Devices, model SpectraMax M5), and cell viability was calculated:

$$\text{Cell viability (\%)} = (A_{\text{(drug treatment group)}} - A_{\text{(blank control)}}) / (A_{\text{(negative control)}} - A_{\text{(blank control)}}) \times 100\%$$

wherein A represents reading of microplate reader.

(6) Experimental results

The results of the virus proliferation inhibition experiment showed that the test compound could effectively inhibit the replication of SARS-CoV-2 virus genome in the infection supernatant at concentrations of 50 μM, 25 μM and 12.5 μM. (Table 3 and Figure 2)

Table 3. Antiviral experiment of test compound (arteannuin B)

| Concentration (μM) | 50 | 25 | 12.5 | 6.25 | 3.13 | 1.56 | Vehicle |
|---|---|---|---|---|---|---|---|
| Number of copies of viral genome (MOI = 0.05) | 176411 ± 32028 | 342654 ± 12201 | 13307075 ± 3309102 | 98310684 ± 8620747 | 99188163 ± 1374175 | 99554711 ± 10871384 | 101677742 ± 41195268 |

The cytotoxicity results showed that the test compound (arteannuin B) had a certain toxicity at concentration of 50μM. The test compound did not change the cell viability at concentrations of 25μM or lower, that was, the test compound had no toxic effect on the cells (Table 4 and Figure 2).

Table 4. Cytotoxicity test of test compound (arteannuin B)

| Concentration (μM) | 100 | 50 | 25 | 12.5 | 6.25 | 3.13 | 1.56 | 0.78 | Vehicle |
|---|---|---|---|---|---|---|---|---|---|
| Cell viability (% of negative control) | 26.24 ± 3.01 | 74.40 ± 4.65 | 95.10 ± 4.32 | 111.44 ± 9.06 | 102.66 ± 4.22 | 94.86 ± 0.61 | 90.63 ± 2.61 | 93.55 ± 1.94 | 100 ± 1.44 |

Example 3: Experiment of reducing viral nucleic acid load in SARS-CoV-2-infected cells by artemether

(1) Drug treatment of virus-infected cells

Vero E6 cells (purchased from ATCC, Cat. No. 1586) were inoculated into a 24-well plate and cultured for 24 hours; then virus infection was performed, specifically, SARS-CoV-2 (2019-nCoV) virus (nCoV-2019BetaCoV/Wuhan/WIV04/2019 strain, provided by Wuhan Institute of Virology, Chinese Academy of Sciences) was diluted to corresponding concentrations with 2% cell maintenance solution (formula: FBS (purchased from Gibco, Cat. No. 16000044) was added to MEM (purchased from Gibco, Cat. No. 10370021) at a volume ratio of 2% to form 2% cell maintenance solution), and then added to the 24-well plate so that each well contained 100 $TCID_{50}$ of virus. Next, artemether (purchased from Selleck Chemicals, Cat. No. S3889) was diluted to corresponding concentrations with 2% cell maintenance solution and added to the corresponding wells, so that the final concentrations of the drug were 200 μM, 100 μM, 50 μM, 25μM, 12.5μM, 6.25μM, respectively, and then the plate was allowed to stand in a 37°C, 5% $CO_2$ incubator for 48 hours, while 2% cell maintenance solution without any test drug was added only in the cell control group.

(2) RNA extraction

RNA extraction kit was purchased from Qiagen, Cat. No. 74106. The consumables (spin column, RNase-free 2mL collection tubes, etc.) and reagents (RLT, RW1, RPE, RNase-free water, etc.) involved in the following RNA extraction steps were all parts of the kit. The following extraction steps were all those recommended by the kit instructions.

1) 100 μL of the supernatant of the test culture plate was taken, added into a nuclease-free EP tube, then added with 350 μL of Buffer RLT, blown and mixed with a pipette gun to make it fully lysed, and then centrifuged to get a supernatant;

2) the supernatant obtained in 1) was added with an equal volume of 70% ethanol, and mixed well;

3) the mixture obtained in the 2) was transferred into an RNase-free spin column, centrifuged at 12,000 rpm for 15s, and the waste liquid was discarded;

4) 700 μL of Buffer RW1 was added to the spin column, centrifuged at 12,000 rpm for 15s to wash the spin column, and the waste liquid was discarded;

5) 500 μL of Buffer RPE was added to the spin column, centrifuged at 12000 rpm for 15s to wash the spin column, and the waste liquid was discarded;

6) 500 μL of Buffer RPE was added to the spin column, centrifuged at 12000 rpm for 2 min to wash the spin column, and the waste liquid was discarded;

7) a new 2mL RNase-free collection tube was used for placement of the spin column, centrifugation was performed at 12,000 rpm for 1 min, the spin column was dried, and then the entire spin column was transferred to the 1.5mL collection tube of step 8);

8) the spin column dried in step 7) was placed in a new 1.5mL collection tube, 30μL of RNase-free water was added to the spin column, centrifuged at 12,000 rpm for 2 minutes, and the eluate contained the corresponding RNA, RNase inhibitor (purchased from NEB company, Cat. No. M0314L) was added, and each RNA concentration was detected with Nano Drop (purchased from Thermo scientific, model Nano Drop One).

(3) RNA reverse transcription

In the experiment, the reverse transcription kit (PrimeScript™ RT reagent Kit with gDNA Eraser, Cat. No. RR047Q) produced by TaKaRa company was used for reverse transcription of RNA, and the steps were as follows.

① gDNA removal: RNA samples were collected from experimental groups, and 1 μg was taken from each sample for reverse transcription. First, 2 μL of 5× gDNA Eraser Buffer was added to the RNA of each experimental group, the reaction system was made up to 10 μL with RNase Free water, then the reaction system was mixed well, and placed in a water bath at 42°C for 2 min to remove the possible gDNA in the sample;

② reverse transcription: appropriate amounts of enzyme, primer Mix and reaction buffer were added to the sample obtained in ①, the volume was made up to 20 μL with RNase Free water, reaction was performed in a water bath at 37°C for 15 minutes, and then performed in a water bath at 85°C for 5 s, thereby obtaining cDNA by transcription.

(4) Real-time PCR

Fluorescence quantitative PCR was used to detect the number of copies per milliliter of the original virus solution.

The reaction system was mixed well using TB Green Premix (Takara, Cat#RR820A), and the amplification reaction and reading were performed in a StepOne Plus Real-time PCR machine (brand: ABI). The number of copies per milliliter of the original virus solution was calculated. The steps were as follows:

① Establishment of standard product: plasmid pMT-RBD (plasmid was provided by Wuhan Institute of Virology, Chinese Academy of Sciences) was diluted to $5\times10^8$ copies/μL, $5\times10^7$ copies/μL, $5\times10^6$ copies/μL, $5\times10^5$ copies/μL, $5\times10^4$ copies/μL, $5\times10^3$ copies/μL, $5\times10^2$ copies/μL. 2 μL of standard or cDNA template was taken and used for qPCR reaction.

② The primer sequences used in the experiment were as follows (all were indicated in the 5'-3' direction):

RBD-qF: CAATGGTTTAACAGGCACAGG (SEQ ID NO: 1)

RBD-qR: CTCAAGTGTCTGTGGATCACG (SEQ ID NO: 2)

③ The reaction procedure was as follows:

pre-denaturation: 95°C for 5 minutes;

cycling parameters: 95°C for 15 seconds, 54°C for 15 seconds, and 72°C for 30 seconds, a total of 40 cycles.

(5) Detection of drug cytotoxicity

The detection of drug cytotoxicity was performed by CCK-8 kit (Beoytime). Specific steps were as follows:

① $1\times10^4$ Vero E6 (ATCC) cells were inoculated into a 96-well plate and cultured at 37°C for 8 hours.
② The test drug was diluted with DMSO to achieve an appropriate stock solution concentration, and then diluted with MEM medium (purchased from Gibco, Cat. No. 10370021) containing 2% FBS (purchased from Gibco, Cat. No. 16000044) to the same concentration as that for the drug treatment. The original medium in the 96-well plate was discarded, 100 μL of the drug-containing MEM medium was added to the cells, and three replicate wells were made for each concentration. Negative controls (adding DMSO and medium to the cell wells without adding drug) and blank controls (no cells, adding DMSO and medium) were set. After the drug was added, the cells were cultured at 37°C for 48 hours.
③ 20 μL of CCK-8 solution (Beoytime) was added to the wells to be tested, mixed gently without generating air bubbles, and continued to incubate at 37°C for 2 hours. $OD_{450}$ was read on a microplate reader (purchased from Molecular Devices, model SpectraMax M5), and cell viability was calculated:

$$\text{Cell viability (\%)} = (A_{\text{(drug treatment group)}} - A_{\text{(blank control)}}) / (A_{\text{(negative control)}} - A_{\text{(blank control)}}) \times 100\%$$

wherein A represents reading of microplate reader.

(6) Experimental results

The results of the virus proliferation inhibition experiment showed that the test compound could effectively inhibit the replication of SARS-CoV-2 virus genome in the infection supernatant at concentrations of 200 μM, 100 μM, 50 μM and 25 μM. (Table 5 and Figure 3)

Table 5. Antiviral experiment of test compound (artemether)

| Concentration (μM) | 200 | 100 | 50 | 25 | 12.5 | 6.25 | Vehicle |
|---|---|---|---|---|---|---|---|
| Number of copies of viral genome (MOI = 0.05) | 250314 ± 19409 | 3326694 ± 150255 | 26849476 ± 4001336 | 32850309 ± 8359067 | 68497905 ± 1231155 | 49305413 ± 11346635 | 66928760 ± 19610420 |

The cytotoxicity results showed that the treatment of the test compound (artemether) did not change the cell viability at all the tested concentrations, that was, the test compound had no toxic effect on the cells at all concentrations (Table 6 and Figure 3).

Table 6. Cytotoxicity test of test compound (artemether)

| Concentration (μM) | 200 | 100 | 50 | 25 | 12.5 | 6.25 | 3.13 | 1.56 | Vehicle |
|---|---|---|---|---|---|---|---|---|---|
| Cell viability (% of negative control) | 132.83 ± 6.46 | 130.18 ± 1.13 | 120.26 ± 5.40 | 118.96 ± 5.25 | 115.49 ± 0.76 | 115.71 ± 0.63 | 103.68 ± 4.60 | 107.12 ± 8.77 | 100 ± 3.21 |

Example 4: Experiment of reducing viral nucleic acid load in SARS-CoV-2-infected cells by artesunate

(1) Drug treatment of virus-infected cells

Vero E6 cells (purchased from ATCC, Cat. No. 1586) were inoculated into a 24-well plate and cultured for 24 hours; then virus infection was performed, specifically, SARS-CoV-2 (2019-nCoV) virus (nCoV-2019BetaCoV/Wuhan/WIV04/2019 strain, provided by Wuhan Institute of Virology, Chinese Academy of Sciences) was diluted to corresponding concentrations with 2% cell maintenance solution (formula: FBS (purchased from Gibco, Cat. No. 16000044) was added to MEM (purchased from Gibco, Cat. No. 10370021) at a volume ratio of 2% to form 2% cell maintenance

solution), and then added to the 24-well plate so that each well contained 100 $TCID_{50}$ of virus. Next, artesunate (purchased from Selleck Chemicals, Cat. No. S2265) was diluted to corresponding concentrations with 2% cell maintenance solution and added to the corresponding wells, so that the final concentrations of the drug were 25 μM, 12.5 μM, 6.25μM, 3.13μM, 1.88μM, 0.94μM, respectively, and then the plate was allowed to stand in a 37°C, 5% $CO_2$ incubator for 48 hours, while 2% cell maintenance solution without any test drug was added only in the cell control group.

(2) RNA extraction

RNA extraction kit was purchased from Qiagen, Cat. No. 74106. The consumables (spin column, RNase-free 2mL collection tubes, etc.) and reagents (RLT, RW1, RPE, RNase-free water, etc.) involved in the following RNA extraction steps were all parts of the kit. The following extraction steps were all those recommended by the kit instructions.

1) 100 μL of the supernatant of the test culture plate was taken, added into a nuclease-free EP tube, then added with 350 μL of Buffer RLT, blown and mixed with a pipette gun to make it fully lysed, and then centrifuged to get a supernatant;

2) the supernatant obtained in 1) was added with an equal volume of 70% ethanol, and mixed well;

3) the mixture obtained in the 2) was transferred into an RNase-free spin column, centrifuged at 12,000 rpm for 15s, and the waste liquid was discarded;

4) 700 μL of Buffer RW1 was added to the spin column, centrifuged at 12,000 rpm for 15s to wash the spin column, and the waste liquid was discarded;

5) 500 μL of Buffer RPE was added to the spin column, centrifuged at 12000 rpm for 15s to wash the spin column, and the waste liquid was discarded;

6) 500 μL of Buffer RPE was added to the spin column, centrifuged at 12000 rpm for 2 min to wash the spin column, and the waste liquid was discarded;

7) a new 2mL RNase-free collection tube was used for placement of the spin column, centrifugation was performed at 12,000 rpm for 1 min, the spin column was dried, and then the entire spin column was transferred to the 1.5mL collection tube of step 8);

8) the spin column dried in step 7) was placed in a new 1.5mL collection tube, 30μL of RNase-free water was added to the spin column, centrifuged at 12,000 rpm for 2 minutes, and the eluate contained the corresponding RNA, RNase inhibitor (purchased from NEB company, Cat. No. M0314L) was added, and each RNA concentration was detected with Nano Drop (purchased from Thermo scientific, model Nano Drop One).

(3) RNA reverse transcription

In the experiment, the reverse transcription kit (PrimeScript™ RT reagent Kit with gDNA Eraser, Cat. No. RR047Q) produced by TaKaRa company was used for reverse transcription of RNA, and the steps were as follows.

① gDNA removal: RNA samples were collected from experimental groups, and 1 μg was taken from each sample for reverse transcription. First, 2 μL of 5× gDNA Eraser Buffer was added to the RNA of each experimental group, the reaction system was made up to 10 μL with RNase Free water, then the reaction system was mixed well, and placed in a water bath at 42°C for 2 min to remove the possible gDNA in the sample;

② reverse transcription: appropriate amounts of enzyme, primer Mix and reaction buffer were added to the sample obtained in ①, the volume was made up to 20 μL with RNase Free water, reaction was performed in a water bath at 37°C for 15 minutes, and then performed in a water bath at 85°C for 5 s, thereby obtaining cDNA by transcription.

(4) Real-time PCR

Fluorescence quantitative PCR was used to detect the number of copies per milliliter of the original virus solution.

The reaction system was mixed well using TB Green Premix (Takara, Cat#RR820A), and the amplification reaction and reading were performed in a StepOne Plus Real-time PCR machine (brand: ABI). The number of copies

per milliliter of the original virus solution was calculated. The steps were as follows:

① Establishment of standard product: plasmid pMT-RBD (plasmid was provided by Wuhan Institute of Virology, Chinese Academy of Sciences) was diluted to $5\times10^8$ copies/μL, $5\times10^7$ copies/μL, $5\times10^6$ copies/μL, $5\times10^5$ copies/μL, $5\times10^4$ copies/μL, $5\times10^3$ copies/μL, $5\times10^2$ copies/μL. 2 μL of standard or cDNA template was taken and used for qPCR reaction.

② The primer sequences used in the experiment were as follows (all were indicated in the 5'-3' direction):

RBD-qF: CAATGGTTTAACAGGCACAGG (SEQ ID NO: 1)

RBD-qR: CTCAAGTGTCTGTGGATCACG (SEQ ID NO: 2)

③ The reaction procedure was as follows:

pre-denaturation: 95°C for 5 minutes;

cycling parameters: 95°C for 15 seconds, 54°C for 15 seconds, and 72°C for 30 seconds, a total of 40 cycles.

(5) Detection of drug cytotoxicity

The detection of drug cytotoxicity was performed by CCK-8 kit (Beoytime). Specific steps were as follows:

① $1\times10^4$ Vero E6 (ATCC) cells were inoculated into a 96-well plate and cultured at 37°C for 8 hours.

② The test drug was diluted with DMSO to achieve an appropriate stock solution concentration, and then diluted with MEM medium (purchased from Gibco, Cat. No. 10370021) containing 2% FBS (purchased from Gibco, Cat. No. 16000044) to the same concentration as that for the drug treatment. The original medium in the 96-well plate was discarded, 100 μL of the drug-containing MEM medium was added to the cells, and three replicate wells were made for each concentration. Negative controls (adding DMSO and medium to the cell wells without adding drug) and blank controls (no cells, adding DMSO and medium) were set. After the drug was added, the cells were cultured at 37°C for 48 hours.

③ 20 μL of CCK-8 solution (Beoytime) was added to the wells to be tested, mixed gently without generating air bubbles, and continued to incubate at 37°C for 2 hours. $OD_{450}$ was read on a microplate reader (purchased from Molecular Devices, model SpectraMax M5), and cell viability was calculated:

$$\text{Cell viability (\%)} = (A_{\text{(drug treatment group)}} - A_{\text{(blank control)}}) / (A_{\text{(negative control)}} - A_{\text{(blank control)}}) \times 100\%$$

wherein A represents reading of microplate reader.

(6) Experimental results

The results of the virus proliferation inhibition experiment showed that the test compound could effectively inhibit the replication of SARS-CoV-2 virus genome in the infection supernatant at concentrations of 25 μM, 12.5 μM and 6.25 μM. (Table 7 and Figure 4)

Table 7. Antiviral experiment of test compound (artesunate)

| Concentratio n (μM) | 25 | 12.5 | 6.25 | 3.13 | 1.88 | 0.94 | Vehicle |
|---|---|---|---|---|---|---|---|
| Number of copies of viral genome (MOI = 0.05) | 70210200 ± 36056435 | 111098018 ± 6636950 | 113126147 ± 29870860 | 170594446 ± 4544786 | 171362509 ± 17037099 | 228511812 ± 62595418 | 222073318 ± 9299837 |

The cytotoxicity results showed that the test compound (artesunate) had a slight toxicity at concentration of 25μM. The test compound did not change the cell viability at concentrations of 12.5μM or lower, that was, the test compound had no toxic effect on the cells (Table 8 and Figure 4).

Table 8. Cytotoxicity test of test compound (artesunate)

| Concentration (μM) | 100 | 50 | 25 | 12.5 | 6.25 | 3.13 | 1.56 | 0.78 | Vehicle |
|---|---|---|---|---|---|---|---|---|---|
| Cell viability (% of negative control) | 21.71 ± 2.56 | 55.44 ± 4.05 | 84.24 ± 1.68 | 93.38 ± 2.49 | 100.64 ± 5.08 | 97.52 ± 3.35 | 99.91 ± 2.50 | 100.09 ± 2.89 | 100 ± 1.32 |

Example 5: Experiment of reducing viral nucleic acid load in SARS-CoV-2-infected cells by dihydroartemisinin

(1) Drug treatment of virus-infected cells

Vero E6 cells (purchased from ATCC, Cat. No. 1586) were inoculated into a 24-well plate and cultured for 24 hours; then virus infection was performed, specifically, SARS-CoV-2 (2019-nCoV) virus (nCoV-2019BetaCoV/Wuhan/WIV04/2019 strain, provided by Wuhan Institute of Virology, Chinese Academy of Sciences) was diluted to corresponding concentrations with 2% cell maintenance solution (formula: FBS (purchased from Gibco, Cat. No. 16000044) was added to MEM (purchased from Gibco, Cat. No. 10370021) at a volume ratio of 2% to form 2% cell maintenance solution), and then added to the 24-well plate so that each well contained 100 $TCID_{50}$ of virus. Next, dihydroartemisinin (purchased from Selleck Chemicals, Cat. No. S2290) was diluted to corresponding concentrations with 2% cell maintenance solution and added to the corresponding wells, so that the final concentrations of the drug were 25 μM, 12.5 μM, 6.25μM, 3.13μM, 1.88μM, 0.94μM, respectively, and then the plate was allowed to stand in a 37°C, 5% $CO_2$ incubator for 48 hours, while 2% cell maintenance solution without any test drug was added only in the cell control group.

(2) RNA extraction

RNA extraction kit was purchased from Qiagen, Cat. No. 74106. The consumables (spin column, RNase-free 2mL collection tubes, etc.) and reagents (RLT, RW1, RPE, RNase-free water, etc.) involved in the following RNA extraction steps were all parts of the kit. The following extraction steps were all those recommended by the kit instructions.

1) 100 μL of the supernatant of the test culture plate was taken, added into a nuclease-free EP tube, then added with 350 μL of Buffer RLT, blown and mixed with a pipette gun to make it fully lysed, and then centrifuged to get a supernatant;

2) the supernatant obtained in 1) was added with an equal volume of 70% ethanol, and mixed well;

3) the mixture obtained in the 2) was transferred into an RNase-free spin column, centrifuged at 12,000 rpm for 15s, and the waste liquid was discarded;

4) 700 μL of Buffer RW1 was added to the spin column, centrifuged at 12,000 rpm for 15s to wash the spin column, and the waste liquid was discarded;

5) 500 μL of Buffer RPE was added to the spin column, centrifuged at 12000 rpm for 15s to wash the spin column, and the waste liquid was discarded;

6) 500 μL of Buffer RPE was added to the spin column, centrifuged at 12000 rpm for 2 min to wash the spin column, and the waste liquid was discarded;

7) a new 2mL RNase-free collection tube was used for placement of the spin column, centrifugation was performed at 12,000 rpm for 1 min, the spin column was dried, and then the entire spin column was transferred to the 1.5mL collection tube of step 8);

8) the spin column dried in step 7) was placed in a new 1.5mL collection tube, 30μL of RNase-free water was added to the spin column, centrifuged at 12,000 rpm for 2 minutes, and the eluate contained the corresponding RNA, RNase inhibitor (purchased from NEB company, Cat. No. M0314L) was added, and each RNA concentration was

detected with Nano Drop (purchased from Thermo scientific, model Nano Drop One).

(3) RNA reverse transcription

In the experiment, the reverse transcription kit (PrimeScript™ RT reagent Kit with gDNA Eraser, Cat. No. RR047Q) produced by TaKaRa company was used for reverse transcription of RNA, and the steps were as follows.

① gDNA removal: RNA samples were collected from experimental groups, and 1 μg was taken from each sample for reverse transcription. First, 2 μL of 5× gDNA Eraser Buffer was added to the RNA of each experimental group, the reaction system was made up to 10 μL with RNase Free water, then the reaction system was mixed well, and placed in a water bath at 42°C for 2 min to remove the possible gDNA in the sample;

② reverse transcription: appropriate amounts of enzyme, primer Mix and reaction buffer were added to the sample obtained in ①, the volume was made up to 20 μL with RNase Free water, reaction was performed in a water bath at 37°C for 15 minutes, and then performed in a water bath at 85°C for 5 s, thereby obtaining cDNA by transcription.

(4) Real-time PCR

Fluorescence quantitative PCR was used to detect the number of copies per milliliter of the original virus solution.

The reaction system was mixed well using TB Green Premix (Takara, Cat#RR820A), and the amplification reaction and reading were performed in a StepOne Plus Real-time PCR machine (brand: ABI). The number of copies per milliliter of the original virus solution was calculated. The steps were as follows:

① Establishment of standard product: plasmid pMT-RBD (plasmid was provided by Wuhan Institute of Virology, Chinese Academy of Sciences) was diluted to $5\times10^8$ copies/μL, $5\times10^7$ copies/μL, $5\times10^6$ copies/μL, $5\times10^5$ copies/μL, $5\times10^4$ copies/μL, $5\times10^3$ copies/μL, $5\times10^2$ copies/μL. 2 μL of standard or cDNA template was taken and used for qPCR reaction.

② The primer sequences used in the experiment were as follows (all were indicated in the 5'-3' direction):

RBD-qF: CAATGGTTTAACAGGCACAGG (SEQ ID NO: 1)

RBD-qR: CTCAAGTGTCTGTGGATCACG (SEQ ID NO: 2)

③ The reaction procedure was as follows:

pre-denaturation: 95°C for 5 minutes;
cycling parameters: 95°C for 15 seconds, 54°C for 15 seconds, and 72°C for 30 seconds, a total of 40 cycles.

(5) Detection of drug cytotoxicity

The detection of drug cytotoxicity was performed by CCK-8 kit (Beoytime). Specific steps were as follows:

① $1\times10^4$ Vero E6 (ATCC) cells were inoculated into a 96-well plate and cultured at 37°C for 8 hours.
② The test drug was diluted with DMSO to achieve an appropriate stock solution concentration, and then diluted with MEM medium (purchased from Gibco, Cat. No. 10370021) containing 2% FBS (purchased from Gibco, Cat. No. 16000044) to the same concentration as that for the drug treatment. The original medium in the 96-well plate was discarded, 100 μL of the drug-containing MEM medium was added to the cells, and three replicate wells were made for each concentration. Negative controls (adding DMSO and medium to the cell wells without adding drug) and blank controls (no cells, adding DMSO and medium) were set. After the drug was added, the cells were cultured at 37°C for 48 hours.
③ 20 μL of CCK-8 solution (Beoytime) was added to the wells to be tested, mixed gently without generating air bubbles, and continued to incubate at 37°C for 2 hours. $OD_{450}$ was read on a microplate reader (purchased from Molecular Devices, model SpectraMax M5), and cell viability was calculated:

$$\text{Cell viability (\%)} = (A_{\text{(drug treatment group)}} - A_{\text{(blank control)}}) / (A_{\text{(negative control)}} - A_{\text{(blank control)}}) \times 100\%$$

wherein A represents reading of microplate reader.

(6) Experimental results

The results of the virus proliferation inhibition experiment showed that the test compound could effectively inhibit the replication of SARS-CoV-2 virus genome in the infection supernatant at concentrations of 25 μM, 12.5 μM and 6.25 μM. (Table 9 and Figure 5)

Table 9. Antiviral experiment of test compound (dihydroartemisinin)

| Concentration (μM) | 25 | 12.5 | 6.25 | 3.13 | 1.88 | 0.94 | Vehicle |
|---|---|---|---|---|---|---|---|
| Number of copies of viral genome (MOI = 0.05) | 23363010 ± 9378355 | 31485734 ± 7672939 | 55425811 ± 11378852 | 90162353 ± 2967186 | 68962319 ± 5241893 | 111625955 ± 25310287 | 81853413 ± 10677031 |

The cytotoxicity results showed that the test compound (dihydroartemisinin) had a slight toxicity at concentration of 12.5μM. The test compound did not change the cell viability at concentrations of 6.25μM or lower, that was, the test compound had no toxic effect on the cells (Table 10 and Figure 5).

Table 10. Cytotoxicity test of test compound (dihydroartemisinin)

| Concentration (μM) | 100 | 50 | 25 | 12.5 | 6.25 | 3.13 | 1.56 | 0.78 | Vehicle |
|---|---|---|---|---|---|---|---|---|---|
| Cell viability (% of negative control) | 2.33 ± 0.38 | 22.18 ± 1.44 | 66.36 ± 2.44 | 88.94 ± 0.49 | 100.38 ± 1.56 | 93.03 ± 7.32 | 98.80 ± 4.72 | 93.93 ± 2.08 | 100 ± 1.98 |

The specific examples described above further describe the purpose, technical solutions and beneficial effects of the present invention in detail. It should be understood that the above-mentioned specific examples are only specific embodiments of the present invention, and are not intended to limit the scope of the present invention. Any modification, equivalent replacement, improvement, etc. made within the spirit and principle of the present invention shall fall into the protection scope of the present invention.

## Claims

1. Use of a compound, or a stereoisomer, a pharmaceutically acceptable salt, a solvate or a hydrate thereof in the manufacture of a medicament for the treatment of a disease or infection caused by a coronavirus (preferably SARS-CoV-2), wherein the compound is one or more selected from the group consisting of artemisinin, arteether, artemether, artemisone, dihydroartemisinin, artesunate, arteannuin B, and artemisinic acid;

    preferably, the disease caused by SARS-CoV-2 is COVID-19;
    preferably, the disease or infection caused by SARS-CoV-2 is a respiratory disease, such as simple infection, mild pneumonia, severe pneumonia, acute respiratory infection, severe acute respiratory infection (SARI), hypoxic respiratory failure, acute respiratory distress syndrome, sepsis, septic shock, etc.;
    preferably, the simple infection includes but is not limited to fever, cough, sore throat, nasal congestion, fatigue, headache, muscle pain or discomfort; preferably, the mild pneumonia includes but is not limited to cough, dyspnea and/or tachypnea; preferably, the severe pneumonia includes but is not limited to, increased respiratory rate, severe respiratory failure or dyspnea, central cyanosis, lethargy, unconsciousness or convulsions, gasp; preferably, the acute respiratory distress syndrome includes but is not limited to pulmonary edema; preferably, the sepsis includes but is not limited to organ dysfunction.

2. The use according to claim 1, wherein the compound is one or more selected from the group consisting of artemisinin, arteannuin B, artemether, artesunate, and dihydroartemisinin;
   preferably, the compound is artemisinin, and has the structure represented by Formula I,

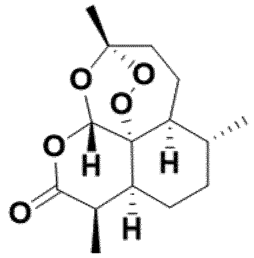

Formula I.

3. Use of a pharmaceutical composition in the manufacture of a medicament for the treatment of a disease or infection caused by a coronavirus (preferably SARS-CoV-2), wherein the pharmaceutical composition comprises a compound, or a stereoisomer, a pharmaceutically acceptable salt, a solvate or a hydrate thereof, and the compound is one or more selected from the group consisting of artemisinin, arteether, artemether, artemisone, dihydroartemisinin, artesunate, arteannuin B, and artemisinic acid, preferably is one or more selected from the group consisting of artemisinin, arteannuin B, artemether, artesunate, and dihydroartemisinin, and more preferably is artemisinin;

preferably, the disease caused by SARS-CoV-2 is COVID-19;
preferably, the disease or infection caused by SARS-CoV-2 is a respiratory disease, such as simple infection, mild pneumonia, severe pneumonia, acute respiratory infection, severe acute respiratory infection (SARI), hypoxic respiratory failure, acute respiratory distress syndrome, sepsis, septic shock, etc.;
preferably, the simple infection includes but is not limited to fever, cough, sore throat, nasal congestion, fatigue, headache, muscle pain or discomfort; preferably, the mild pneumonia includes but is not limited to cough, dyspnea and/or tachypnea; preferably, the severe pneumonia includes but is not limited to, increased respiratory rate, severe respiratory failure or dyspnea, central cyanosis, lethargy, unconsciousness or convulsions, gasp; preferably, the acute respiratory distress syndrome includes but is not limited to pulmonary edema; preferably, the sepsis includes but is not limited to organ dysfunction;
preferably, the pharmaceutical composition further comprises benflumetol;
preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

4. Use of a compound, or a stereoisomer, a pharmaceutically acceptable salt, a solvate or a hydrate thereof in the manufacture of a medicament as an inhibitor of a coronavirus (preferably SARS-CoV-2), wherein the compound is one or more selected from the group consisting of artemisinin, arteether, artemether, artemisone, dihydroartemisinin, artesunate, arteannuin B, and artemisinic acid.

5. The use according to claim 4, wherein the compound is one or more selected from the group consisting of artemisinin, arteannuin B, artemether, artesunate, and dihydroartemisinin;
preferably, the compound is artemisinin, and has the structure represented by Formula I,

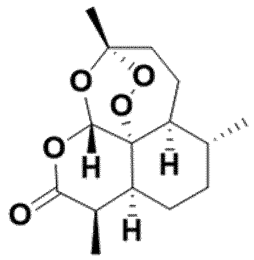

Formula I.

6. Use of a pharmaceutical composition in the manufacture of a medicament as an inhibitor of a coronavirus (preferably SARS-CoV-2), wherein the pharmaceutical composition comprises a compound, or a stereoisomer, a pharmaceutically acceptable salt, a solvate or a hydrate thereof, the compound is one or more selected from the group consisting

of artemisinin, arteether, artemether, artemisone, dihydroartemisinin, artesunate, arteannuin B, and artemisinic acid, preferably is one or more selected from the group consisting of artemisinin, arteannuin B, artemether, artesunate, and dihydroartemisinin, and more preferably is artemisinin;

preferably, the pharmaceutical composition further comprises benflumetol;
preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

**7.** Use of a compound, or a stereoisomer, a pharmaceutically acceptable salt, a solvate or a hydrate thereof in the manufacture of a medicament for inhibiting the replication or reproduction of a coronavirus (preferably SARS-CoV-2) in a cell (e.g., mammalian cell), wherein the compound is one or more selected from the group consisting of artemisinin, arteether, artemether, artemisone, dihydroartemisinin, artesunate, arteannuin B, and artemisinic acid.

**8.** The use according to claim 7, wherein the compound is one or more selected from the group consisting of artemisinin, arteannuin B, artemether, artesunate, and dihydroartemisinin;
preferably, the compound is artemisinin, and has the structure represented by Formula I,

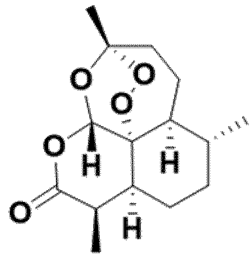

Formula I.

**9.** Use of a pharmaceutical composition in the manufacture of a medicament for inhibiting the replication or reproduction of a coronavirus (preferably SARS-CoV-2) in a cell (e.g., mammalian cell), wherein the pharmaceutical composition comprises a compound, or a stereoisomer, a pharmaceutically acceptable salt, a solvate or a hydrate thereof, the compound is one or more selected from the group consisting of artemisinin, arteether, artemether, artemisone, dihydroartemisinin, artesunate, arteannuin B, and artemisinic acid, preferably is one or more selected from the group consisting of artemisinin, arteannuin B, artemether, artesunate, and dihydroartemisinin, and more preferably is artemisinin;

preferably, the pharmaceutical composition further comprises benflumetol;
preferably, the pharmaceutical composition further comprises a pharmaceutically acceptable carrier or excipient, specifically, the pharmaceutical composition is a solid preparation, an injection, an external preparation, a spray, a liquid preparation, or a compound preparation.

**10.** The use according to claim 7 or 9, wherein the mammal comprises bovine, equine, ovine, porcine, canine, feline, rodent, primate, such as human, cat, dog or pig.

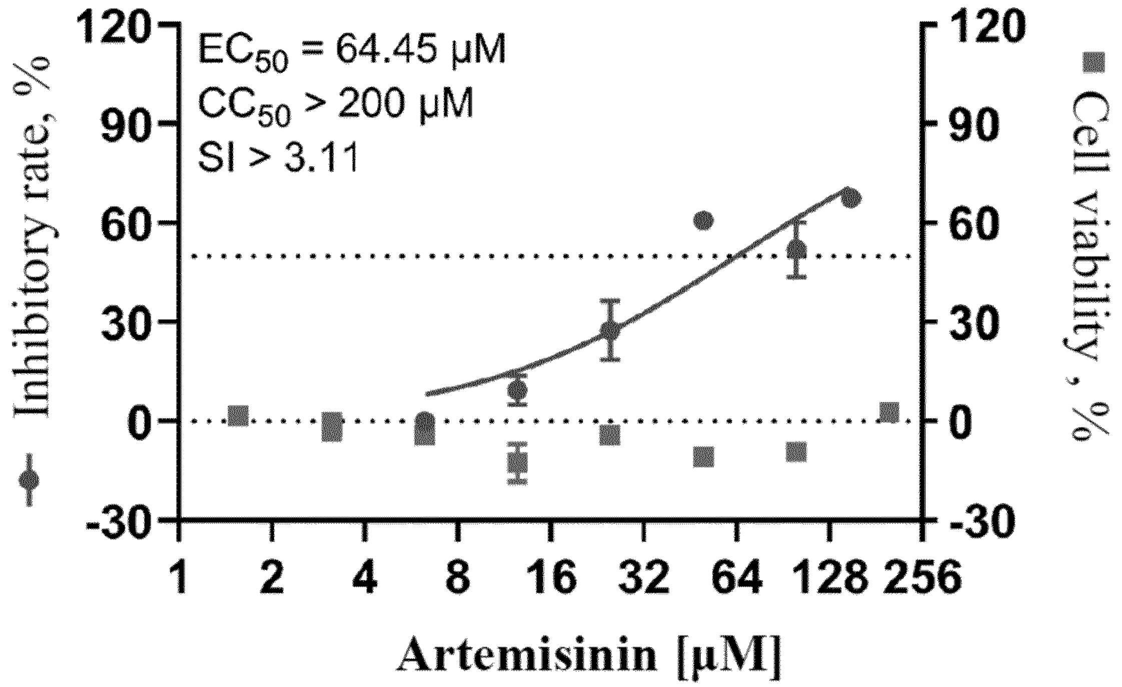


Figure 1

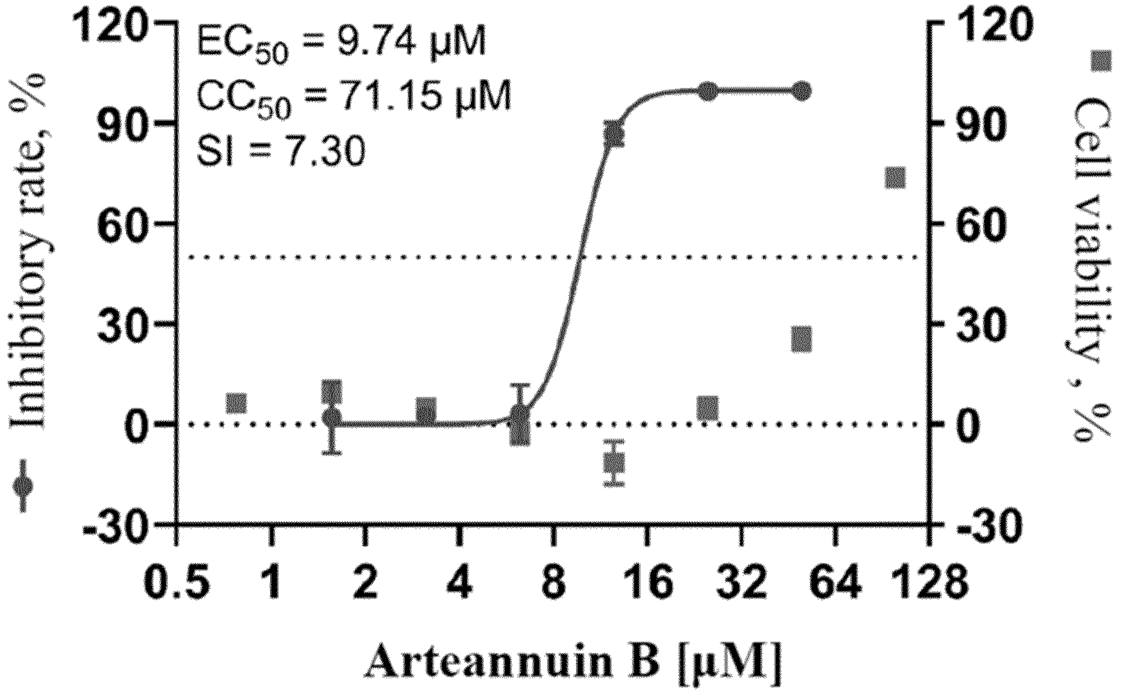


Figure 2

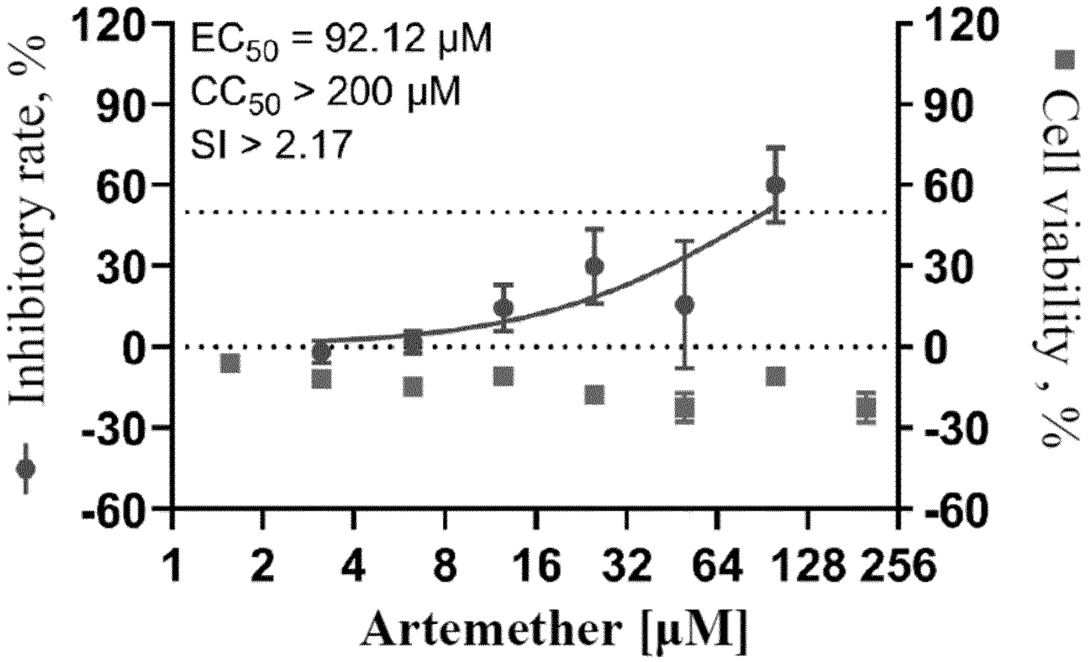


Figure 3

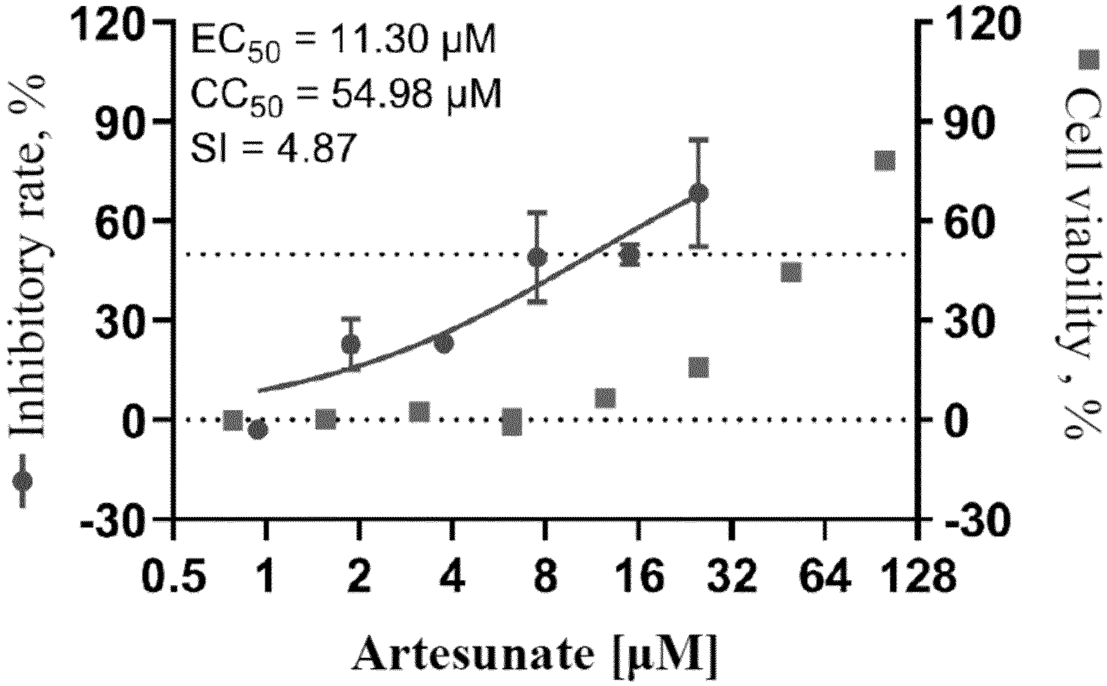


Figure 4

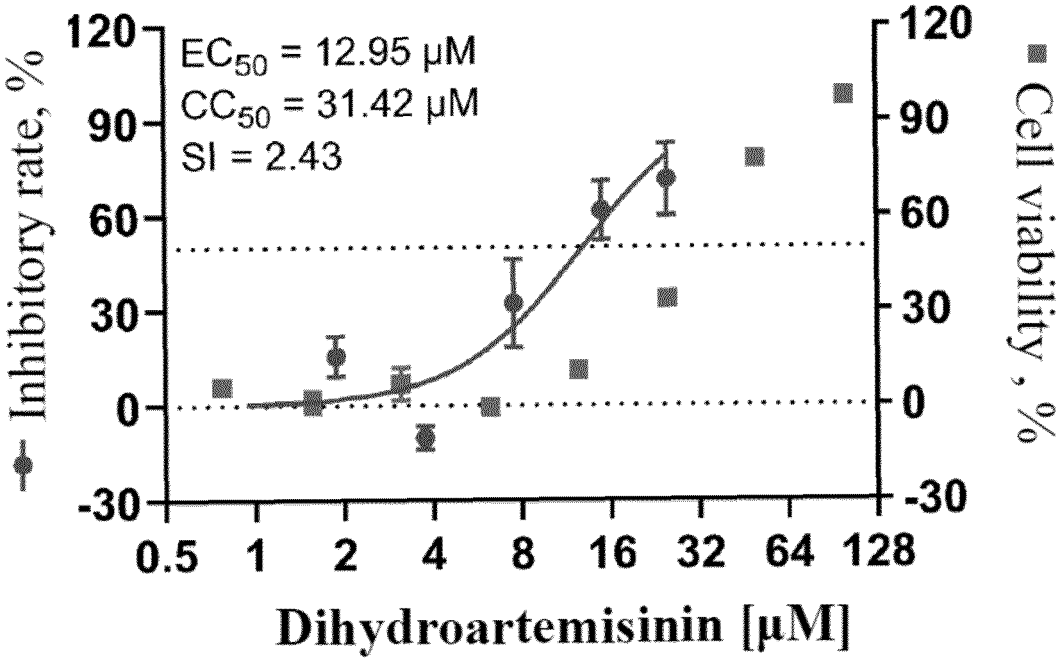
EC50 = 12.95 μM
CC50 = 31.42 μM
SI = 2.43
Inhibitory rate, %
Cell viability , %
120
90
60
30
0
-30
0.5
1
2
4
8
16
32
64
128
Dihydroartemisinin [μM]

Figure 5

**INTERNATIONAL SEARCH REPORT**

International application No. **PCT/CN2021/107772**

**A. CLASSIFICATION OF SUBJECT MATTER**

A61K 31/366(2006.01)i; A61K 31/357(2006.01)i; A61K 31/541(2006.01)i; A61K 31/365(2006.01)i; A61K 31/19(2006.01)i; A61K 31/137(2006.01)i; A61K 9/00(2006.01)i; A61P 31/14(2006.01)i; A61P 11/00(2006.01)i

According to International Patent Classification (IPC) or to both national classification and IPC

**B. FIELDS SEARCHED**

Minimum documentation searched (classification system followed by classification symbols)

A61K, A61P

Documentation searched other than minimum documentation to the extent that such documents are included in the fields searched

Electronic data base consulted during the international search (name of data base and, where practicable, search terms used)

CNTXT; CNABS; CJFD; CNKI; VEN; STN: COVID, 青蒿乙素, 青蒿酸, 蒿甲醚, 青蒿酮, 青蒿素, 冠状病毒, 蒿乙醚, 青蒿琥酯, +artemisinin, artesunate, arteannium, coronavirus, sars, mers, nCov, COVID

**C. DOCUMENTS CONSIDERED TO BE RELEVANT**

| Category* | Citation of document, with indication, where appropriate, of the relevant passages | Relevant to claim No. |
|---|---|---|
| PX | CN 111686096 A (ACAD OF MILITARY SCIENCES PLA CHINA ACAD OF MILITARY MEDICAL SCIENCES) 22 September 2020 (2020-09-22)<br>claims 3, 6, 9-10 | 1-10 |
| PX | CN 111803491 A (ACAD OF MILITARY SCIENCES PLA CHINA ACAD OF MILITARY MEDICAL SCIENCES) 23 October 2020 (2020-10-23)<br>claims 1-10 | 1-10 |
| X | Tuğçenur Uzun and Orcun Toptas. "“Artesunate:could be an alternative drug to chloroquine in COVID-19 treatment?”"<br>《*Chin Med*》, Vol. 15, No. 54, 28 May 2020 (2020-05-28),<br>abstract, page 2, left-hand column, paragraph 2 - page 3, left-hand column, paragraph 1 | 1-10 |
| X | Moussa Sehailia and Smain Chemat. "“Antimalarial-agent artemisinin and derivatives portray more potent binding to Lys353 and lys31-binding hotspots of SARS-CoV-2 spike protein than hydroxychloroquine:potential repurposing of artenimol for COVID-19”"<br>《*JOURNAL OF BIOMOLECULAR STRUCTURE AND DYNAMICS*》,<br>22 July 2020 (2020-07-22),<br>abstract, page 2 left-hand column paragraph 2, page 5 left-hand column paragraph 2-page 7 right-hand column paragraph 1, table 1 | 1-10 |

☑ Further documents are listed in the continuation of Box C. ☑ See patent family annex.

* Special categories of cited documents:
- “A” document defining the general state of the art which is not considered to be of particular relevance
- “E” earlier application or patent but published on or after the international filing date
- “L” document which may throw doubts on priority claim(s) or which is cited to establish the publication date of another citation or other special reason (as specified)
- “O” document referring to an oral disclosure, use, exhibition or other means
- “P” document published prior to the international filing date but later than the priority date claimed
- “T” later document published after the international filing date or priority date and not in conflict with the application but cited to understand the principle or theory underlying the invention
- “X” document of particular relevance; the claimed invention cannot be considered novel or cannot be considered to involve an inventive step when the document is taken alone
- “Y” document of particular relevance; the claimed invention cannot be considered to involve an inventive step when the document is combined with one or more other such documents, such combination being obvious to a person skilled in the art
- “&” document member of the same patent family

| Date of the actual completion of the international search | Date of mailing of the international search report |
|---|---|
| **26 September 2021** | **14 October 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

INTERNATIONAL SEARCH REPORT

International application No.
**PCT/CN2021/107772**

**C. DOCUMENTS CONSIDERED TO BE RELEVANT**

| Category* | Citation of document, with indication, where appropriate, of the relevant passages | Relevant to claim No. |
|---|---|---|
| X | 刘佳梅 等 (LIU, Jiamei et al.). "“青蒿素及其衍生物用于治疗新型冠状病毒肺炎的探讨” (Discussion on artemisinin and its derivatives for treatment of COVID-19)" *《药物评价研究》 (Drug Evaluation Research),* Vol. 43, No. 4, 30 April 2020 (2020-04-30), abstract, page 608 left-hand column, section 2-page 610 right-hand column, paragraph 2 | 1-10 |

Form PCT/ISA/210 (second sheet) (January 2015)

**INTERNATIONAL SEARCH REPORT**
**Information on patent family members**

International application No.
**PCT/CN2021/107772**

| Patent document cited in search report | Publication date (day/month/year) | Patent family member(s) | Publication date (day/month/year) |
|---|---|---|---|
| CN 111686096 A | 22 September 2020 | None | |
| CN 111803491 A | 23 October 2020 | None | |

Form PCT/ISA/210 (patent family annex) (January 2015)